Europäisches Patentamt

European Patent Office

Office européen des brevets

(11) Publication number: **0 129 268**
**B1**

## (12) EUROPEAN PATENT SPECIFICATION

(45) Date of publication of patent specification: 04.10.89

(21) Application number: 84200735.3

(22) Date of filing: 21.05.84

(51) Int. Cl.⁴: **C 12 N 15/00, C 12 P 21/02,**
**C 07 H 21/04, C 12 N 1/16 //**
**(C12N1/16, C12R1:645)**

(54) Improvements in the expression of newly introduced genes in yeast cells.

(30) Priority: 19.05.83 EP 83200713

(43) Date of publication of application:
27.12.84 Bulletin 84/52

(45) Publication of the grant of the patent:
04.10.89 Bulletin 89/40

(84) Designated Contracting States:
AT BE CH DE FR GB IT LI NL SE

(56) References cited:
EP-A-0 001 931
EP-A-0 046 039
EP-A-0 054 330
EP-A-0 057 350
EP-A-0 060 057
EP-A-0 073 635
EP-A-0 077 689
EP-A-0 089 666
EP-A-0 096 430
EP-A-0 096 910

The file contains technical information
submitted after the application was filed and
not included in this specification

(73) Proprietor: UNILEVER NV
Burgemeester s'Jacobplein 1 P.O. Box 760
NL-3000 DK Rotterdam (NL)
(84) BE CH DE FR IT LI NL SE AT

(73) Proprietor: UNILEVER PLC
Unilever House Blackfriars P.O. Box 68
London EC4P 4BQ (GB)
(84) GB

(72) Inventor: Edens, Luppo
Albert Schweitzerdreef 29
NL-3146 AA Maassluis (NL)
Inventor: Russell, Stephen William
Josef Israelslaan 13
NL-3141 KC Maassluis (NL)
Inventor: Visser, Christiaan
Minoeserf 77
NL-2907 VJ Capelle a/d IJssel (NL)
Inventor: Verrips, Cornelis Theodorus
Hagedoorn 18
NL-3142 KB Maassluis (NL)

(74) Representative: van der Toorren, Johannes, Drs.
et al
UNILEVER N.V. Patent Division Postbus 137
NL-3130 AC Vlaardingen (NL)

Courier Press, Leamington Spa, England.

(56) References cited:

DRUGS DEVELOPMENT RESEARCH 1, 1981;
L.MILLER et al.: "Synthesis of biologically
active proteins by recombinant DNA
technology", pp. 435-454

JOURNAL OF BIOLOGICAL CHEMISTRY, vol.
256, no. 3, 10 Feb 1981; M.J.HOLLAND et al.:
"The primary structures of two yeast enolase
genes", pp. 1385-1395

JOURNAL OF BIOLICAL CHEMISTRY, vo. 255,
1980; pp. 2596-2605

## Description

The present invention relates to improvements in the expression of newly introduced genes in yeast cells.

In particular the invention relates to a DNA sequence capable of initiating transcription by yeast RNA polymerase II which includes at least part of the regulon region of one of the GAPDH genes of S. cerevisiae. (DNA = DeoxyriboNucleic Acid; RNA = RiboNucleic Acid; GAPDH = GlycerAldehyde-3-Phosphate DeHydrogenase; mRNA = messenger RNA).

Said DNA may be combined with a DNA sequence capable of both termination of the transcription by yeast RNA polymerase II and effecting polyadenylation of the mRNA, which includes at least part of the termination/polyadenylation region belonging to one of the GAPDH genes of S. cerevisiae.

The invention also relates to a larger rDNA sequence which contains at least the above-indicated regulon region of one of the GAPDH genes of S. cerevisiae and one or more structural genes different from the GAPDH genes of S. cerevisiae, which DNA sequence can be inserted into a recombinant DNA plasmid or into a yeast chromosome in order to transform yeasts so that they become able to produce the desired proteins encoded by the structural genes.

Finally, the invention relates to a process for preparing a protein by cultivating a yeast containing the above-mentioned larger rDNA sequences under conditions whereby the protein is formed and isolating the protein from that yeast culture, as well as the proteins produced by such a process.

Background of the Invention

Developments in recombinant DNA (= rDNA) technology have made it possible to isolate or synthesize specific genes or portions thereof from higher organisms such as man, animals and plants, and to transfer these genes or gene fragments to microorganisms such as bacteria or yeasts. The transferred gene is replicated and propagated as the transformed microorganism replicates. As a result, the transformed microorganism may become endowed with the capacity to make whatever protein the transferred gene or gene fragment encodes whether it is an enzyme, a hormone, an antigen, an antibody, or a portion thereof. The microorganism passes on this capability to its progeny, so that in effect, the transfer has resulted in a new microbial strain, having the described capability.

A basic fact underlying the application of this technology for practical purposes is that DNA of all living organisms, from microbes to man, is chemically similar, being composed of the same four nucleotides. For example, the same nucleotide sequence which codes for the amino acid sequence specifying preprochymosin in stomach cells of newborn mammals, will, when transferred to a microorganism, be recognized as coding for the same amino acid sequence.

The basic constituents of the recombinant DNA technology are formed by:

i) the gene encoding the protein of interest.

ii) a vector (plasmid) in which the new gene has to be inserted to guarantee stable replication and a high level of expression of the gene.

iii) a suitable host microorganism in which the vector carrying the new gene can be introduced.

Depending on the nature of the protein to be synthesized, the industrial application of this protein and the technically possible fermentation and purification procedures, the plasmid vector and the host organism have to be selected. In most cases the selection of a host organism which is unsuspected with regard to the production of toxic substances, i.e. a microorganism mentioned in the GRAS (Generally Recognized As Safe) list, will be highly important. However, only very few of these GRAS-microorganisms meet all requirements asked for by the application of either the recombinant DNA or the fermentation technology. A selection on the basis of these positive criteria shows that, at this moment, certain yeast species, notably Saccharomyces, Kluyveromyces, Debaryomyces, Hansenula, Candida, Torulopsis and Rhodotorula, can be regarded as very promising host organisms for genetically engineered DNA molecules.

In the present invention use is made of recombinant DNA, molecular, biological and chemical techniques to construct plasmid vectors that can be stably maintained within yeasts and, most importantly, contain the appropriate regulons to bring about a high level of expression of newly introduced genes.

Several plasmid vectors are known nowadays which can be used for the transformation of the yeast Saccharomyces cerevisiae [C. P. Hollenberg, Current Topics in Microb. and Immunol. 96, 119—144 (1982) and A. Hinnen and B. Meyhack, Current Topics in Microb. and Immunol. 96, 101—117 (1982)]. These vectors rely on either autonomous replication sequences (ARS) isolated from the chromosomal DNA of particular yeast species or the replicating ability of the 2 micron DNA plasmid found in Saccharomyces cerevisiae to maintain the vector and the inserted gene within the host cell. Additionally these yeast vectors contain a marker by which transformants can be selected. Examples of such markers are the leu 2 gene [A. Hinnen et al., Proc. Natl. Acad. Sci. USA, 75, 1929—1933 (1978)], the trp 1 gene [K. Struhl et al., Proc. Natl. Acad. Sci. USA, 76, 1035—1039 (1979)], the lactase gene [R. C. Dickson, Gene, 10, 347—356 (1980)], and genes which confer resistance of the host cell against certain antibiotics.

The stability of AR-sequences in S. cerevisiae or (K)AR sequences in Kluyveromyces lactis or K. fragilis is not always sufficient for the development of a reliable fermentation process using these yeasts. Therefore, integration of the foreign structural gene into the chromosome(s) of the new host cell can be very important for the industrial application of rDNA-containing yeasts.

Seen from an economic point of view not only the stability of the inserted gene within the yeast cell is important but also the efficiency with which this gene is expressed as protein. Based on todays knowledge, the main routes to achieve high levels of expression of a newly inserted gene include:

integration of the structural gene downstream of a promoter (RNA-initiation) site which can effect a high transcription frequency of the gene. Ideal is that the promoter activity is inducible, i.e. can be switched on or off depending upon a temperature shift or the presence of an inducer in the growth medium. Potent promoters operating in yeasts are those responsible for transcription of the genes encoding glycolytic enzymes. Experimental work done by Maitra and Lobo [J. Biol. Chem., 246, 489—499 (1971)] suggests furthermore that some of these promoters are highly inducible. However a serious difficulty with regard to the isolation of such promoters is that up to now little is known about the nucleotide sequences which confer regulation and full promoter activity on the DNA fragment

integration of an RNA (RiboNucleic Acid) termination signal downstream of the structural gene. Owing to the presence of such a termination signal, transcription of the structural gene cannot interfere with the transcription of adjacent operons. Moreover transcription seems to be more efficient [K. S. Zaret and F. Sherman, Cell, 28, 563—573 (1982)] and polyadenylation of the messenger is likely to occur correctly, resulting in a more stable mRNA (messenger RNA) population. However, up to now exact data on nucleotide sequences required for termination of transcription in yeasts are not available.

the presence of a nucleotide sequence flanking the AUG codon in the RNA-molecule which is optimal for protein synthesis initiation. According to published data [M. Kozak, Nucleic Acids Res. 9, 5233—5252 (1981)] the positions −3 and +4, N X X A U G N are highly conserved (A or C at −3 and G at +4), which observation suggests a role for these nucleotides in the recognition of the AUG codon as a translation start point by the ribosome. Although one might expect an efficient yeast promoter to contain either an A or C as nucleotide at the −3 position, the nucleotide at the +4 position forms part of the coding sequence and is therefore dependent upon the nature of the gene to be inserted downstream of the promoter. This indicates that it will be difficult to fulfil this condition in all cases.

the copy number of the vector within the host cell. In most cases high copy numbers will lead to higher mRNA levels and, therefore, to higher expression levels of a gene. In S. cerevisiae vectors containing the 2 micron DNA replication origin can reach copy-numbers as high as 50. This is considerably more than can be reached by for instance integrating vectors or vectors containing autonomously replicating sequences (ARS) [K. Struhl et al., Proc. Natl. Acad. Sci. USA, 76, 1035—1039 (1979) and A. J. Kingsman et al., Gene, 7, 141—152 (1979)]. However, in yeast species other than Saccharomyces, 2 micron DNA has not yet been found, suggesting that its replication origin will be functional in a very limited number of yeast species only. Experimental data obtained so far show that the 2 micron replication origin is functional in Schizosaccharomyces pombe [D. Beach and P. Nurse, Nature, 290, 140—142 (1981)], but not in Kluyveromyces lactis (G. Das and C. P. Hollenberg, Curr. Genet. 5, 123—128 (1982)].

Therefore the transformation of yeasts belonging to genera other than Saccharomyces or Schizosaccharomyces will be dependent in most cases upon the availability of other DNA replication origins such as ARS isolated from the organism to be transformed or upon the integration of the foreign gene into the yeast genome:

a codon use of the gene which is optimal for the host organism used. Results obtained with the yeast S. cerevisiae show a strong correlation between the abundance of certain tRNA (transfer RNA) species and the occurrence of the respective codons in its protein genes. Therefore, optimal expression of for instance the bovine preprochymosin gene or the plant preprothaumatin gene in S. cerevisiae would require a chemical synthesis of both genes with a codon population which correlates with these abundant yeast tRNA species.

an additional factor which might influence the translation of a gene is the presence of a DNA sequence encoding a so-called signal sequence. These signal sequences are in most cases hydrophobic N-terminal protein extensions which are often involved in the process of cotranslational secretion of the protein through a membrane. In the present specification new data are shown obtained with the expression of the preprothaumatin gene in yeast, indicating that when the DNA sequence encoding the signal protein is removed from the gene, expression of the gene is reduced by a few orders of magnitude.

At present the number of known yeast species which can serve as a host for recombinant DNA molecules is limited to Saccharomyces cerevisiae and Schizosaccharomyces pombe.

Summary of the Invention

Therefore, a need exists for additional yeast species with other metabolic properties and other nutritional demands, so that the field to which recombinant DNA technology can be applied on a commercial level will be broadened. However, the use of new yeast species requires suitable DNA replication origins to guarantee a stable replication of the vector molecule in the new host as well as an appropriate expression system for newly inserted DNA. The present invention provides an expression system, the use of which is not restricted to S. cerevisiae but which can function in other yeast species as well. In order to achieve this the RNA initiation/regulation and the RNA termination/polyadenylation signals of a glyceraldehyde-3-phosphate dehydrogenase (GAPDH) gene of S. cerevisiae were isolated.

Besides the fact that the RNA initiation site of a GAPDH gene is a very efficient promoter in S. cerevisiae

# EP 0 129 268 B1

[M. J. Holland *et al*. Biochemistry *17*, 4900—4907 (1978)] the promoter according to the present invention was isolated and applied after it was realized that GAPDH is a metabolic key enzyme in many different organisms, suggesting a certain conservatism during evolution with regard to the nucleotide sequence regulating its expression. On this basis further experiments were carried out. It was observed (i) that the isolated and radioactively labelled GAPDH regulon fragment hybridized with colonies of various yeast species, (ii) the *S. cerevisiae* GAPDH regulon expressed foreign genes in *K. lactis* efficiently and (iii) the larger regulon region of about 850 nucleotides was more effective than the smaller regulon region of about 280 nucleotides published by Holland J. P. and Holland M. J. in J. Biol. Chem. *255*, 2596—2605 (1980). These new findings gave us confidence that the regulon isolated will prove to be useful in the expression of foreign DNA in a number of other yeast species.

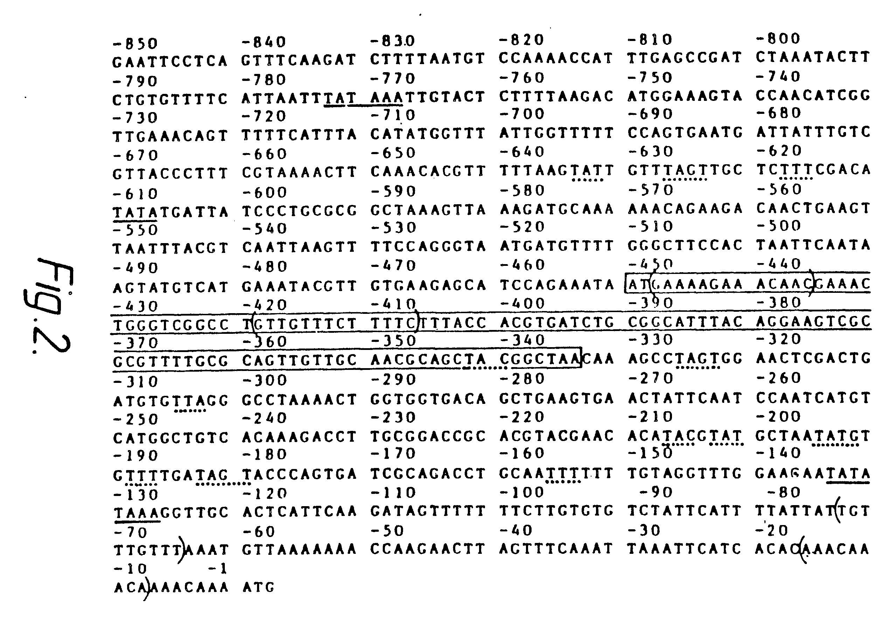

The present invention provides a DNA sequence capable of initiating transcription by yeast RNA polymerase II which includes at least part of the regulon region of one of the GAPDH genes of *S. cerevisiae*, characterized in that it comprises a DNA sequence essentially as given in Fig. 2, and wherein the regulon region is optionally modified to include at least one restriction enzyme cleavage site, to facilitate manipulation of the nucleotide sequence region for protein synthesis initiation.

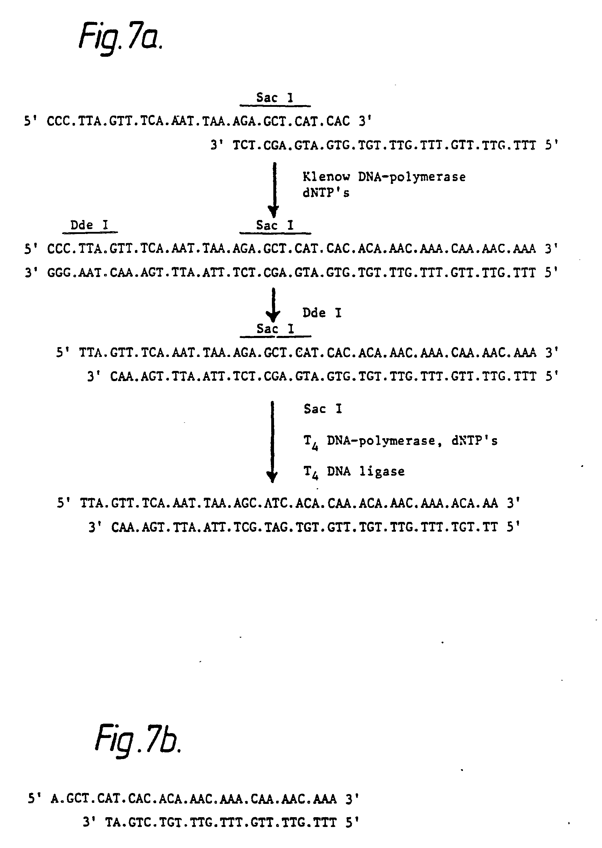

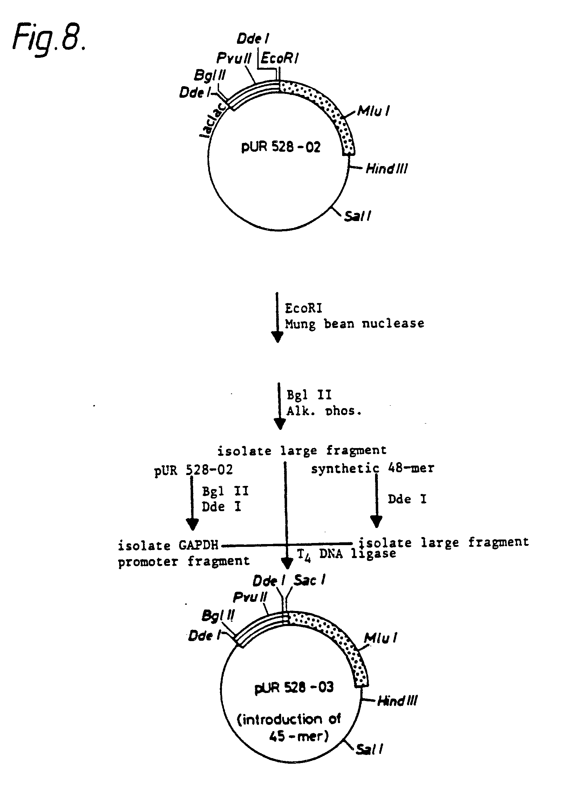

An example of a modification of the regulon is given in item 4 and Figs. 7A and 8, where the introduction of a Sac I site is described. Although in Fig. 2 only one specific DNA sequence is described, it will be clear to the expert that modifications of this DNA sequence, either by replacement of one or more nucleotides or by addition or deletion of one or more nucleotides, which modifications do not impair the properties of the regulon region given in Fig. 2, are within the realm of the invention.

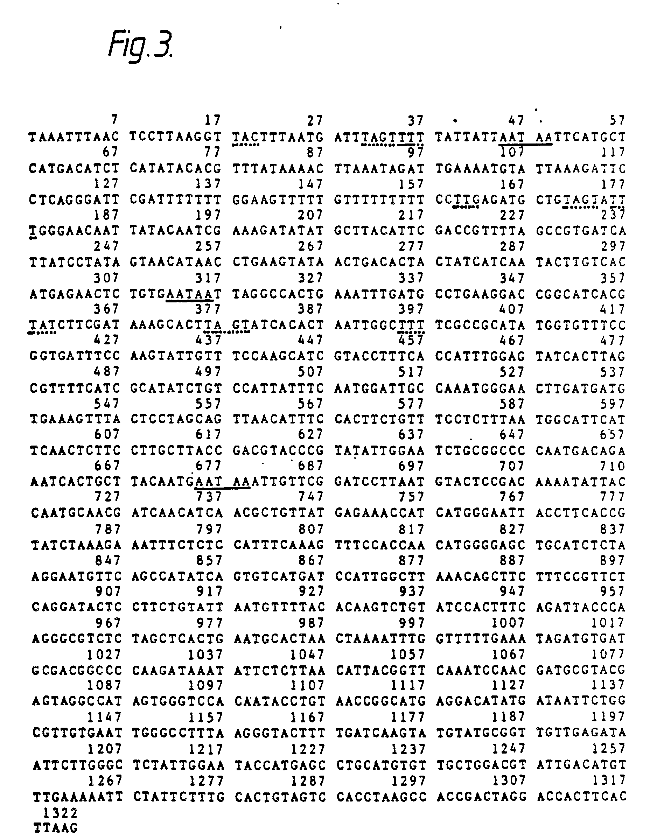

In a preferred embodiment the DNA sequence capable of initiating transcription is combined with a DNA sequence capable of both termination of the transcription by yeast RNA polymerase II and effecting polyadenylation of the mRNA, which includes at least part of the termination/polyadenylation region belonging to one of the GAPDH genes of *S. cerevisiae*, characterised in that it comprises a DNA sequence essentially as given in Fig. 3.

Indications have been obtained that the presence of such DNA sequence is favourable for the expression of structural genes in yeasts. The presence of such DNA sequence seems to be particularly advantageous for incorporating the rDNA in the genome of a yeast cell.

The invention also provides a DNA sequence, which can be inserted into a recombinant DNA plasmid or into a yeast chromosome, comprising:

(a) a DNA sequence essentially as given in Fig. 2, and

(b) one or more structural genes different from the GAPDH genes of *S. cerevisiae*, and at least two of features (c)—(f),

(c) one or more specific DNA sequences capable of terminating the transcription by yeast RNA polymerase II and effecting polyadenylation of the mRNA, and/or

(d) one or more selection markers, and/or

(e) either one or more nucleotide sequences allowing a stable insertion in a chromosome of yeasts or one or more DNA sequences which regulate DNA replication in yeasts belonging to the genus *Saccharomyces* or to the genus *Kluyveromyces*, and/or

(f) a DNA sequence encoding a signal polypeptide of not more than 30 amino acid residues assisting the translocation of proteins, which DNA sequence is situated upstream of and in the same reading frame as the structural gene.

In particular, the structural gene of (b) encodes thaumatin or chymosin, or their various allelic or modified forms, which modified forms do not impair the sweet-tasting properties of thaumatin or the milk-clotting properties of chymosin, respectively, or precursors of these thaumatin-like or chymosin-like proteins, since such DNA sequence will assist in giving an increased expression of these genes.

A preference exists to use a DNA sequence essentially as given in Fig. 3 as the specific DNA sequence of (c), since this seems more adapted to yeast than other termination/polyadenylation regions.

The DNA sequence of (f) encoding a signal polypeptide can be selected from the group consisting of DNA sequences encoding

(a) the signal polypeptide translocating *S. cerevisiae* invertase, namely Met.Leu.Leu.Gln.Ala.Phe.Leu.Phe.Leu.Leu.Ala.Gly.Phe.Ala.Ala.Lys.Ile.Ser.Ala;

(b) the signal polypeptide translocating *S. cerevisiae* acid phosphatase, namely Met.Phe.Lys.Ser.Val.Val.Tyr.Ser.Ile.Leu.Ala.Ala.Ser.Leu.Ala.Asn.Ala;

(c) the signal polypeptide of unmatured forms, of thaumatin-like proteins, namely Met.Ala.Ala.Thr.Thr.Cys.Phe.Phe.Phe.Leu.Phe.Pro.Phe.Leu.Leu.Leu.Leu.Thr.Leu.Ser.Arg.Ala;

(d) the signal polypeptide of unmatured forms of chymosin-like proteins, namely Met.Arg.Cys.Leu.Val.Val.Leu.Leu.Ala.Val.Phe.Ala.Leu.Ser.Gln.Gly; and

5

(e) two consensus signal polypeptides, namely Met.Ser.Lys.Ala.Ala.Leu.Ala.Phe.Ile.Ala.Phe.Val.Ile.Val.Leu.Ile.Val.Asn.Ala and Met.Ser.Lys.Phe.Val.Ile.Val.Leu.Ile.Val.Ala.Ala.Leu.Ala.Phe.Ile.Ala.Asn.Ala.

In order to make the conditions for expression as good as possible it is advocated to modify the structural gene of (b) and/or the signal polypeptide-encoding DNA sequence of (f) such that the codons are codons preferred by yeasts. Following the teaching of J. P. Holland and M. J. Holland (J. Biol. Chem. *255*, 2596—2605 (1980]) the preferred codons are:

| | | | |
|---|---|---|---|
| GCC or GCT | alanine | TTG | leucine |
| AGA | arginine | AAG | lysine |
| AAC | asparagine | ATG | methionine |
| GAC or GAT | aspartic acid | TTC | phenylalanine |
| TGT | cysteine | CCA | proline |
| CAA | glutamine | TCC or TCT | serine |
| GAA | glutamic acid | ACC or ACT | threonine |
| GGT | glycine | TGG | tryptophan |
| CAC | histidine | TAC | tyrosine |
| ATC or ATT | isoleucine | GTC or GTT | valine |

The DNA sequences described above are not a purpose in themselves. They will be used in a process for preparing yeasts containing these DNA sequences by introducing these rDNA sequences into *Saccharomyces, Kluyveromyces, Hansenula, Candida* or *Torulopsis* yeasts, either in the form of plasmids or by incorporation in the yeast genome.

The invention further provides yeasts containing such rDNA sequences, either in the form of a plasmid or incorporated in the yeast genome and their use in a process for preparing a protein by cultivation of such yeast, whereby the rDNA sequence incorporated in the yeast contains a structural gene encoding that protein or a precursor thereof, which precursor can form the relevant protein during processing.

Finally, the invention provides the proteins produced by this novel process.

Several constructions in which the GAPDH promoter/regulation region was combined with structural genes encoding either preprothaumatin or preprochymosin or some of the various maturation products have been made and synthesis of thaumatin-like proteins in yeast have been demonstrated. Preferred constructions contained structural genes encoding either preprothaumatin or prethaumatin. To improve the expression yield of said genes, the original codons can be replaced by codons which are abundantly present in highly expressed genes. Moreover, the DNA sequence encoding the signal sequence of preprochymosin can be replaced by DNA sequences encoding signal sequences of products excreted by yeasts, such as the signal sequences invertase and acid phosphatase produced by *S. cerevisiae*.

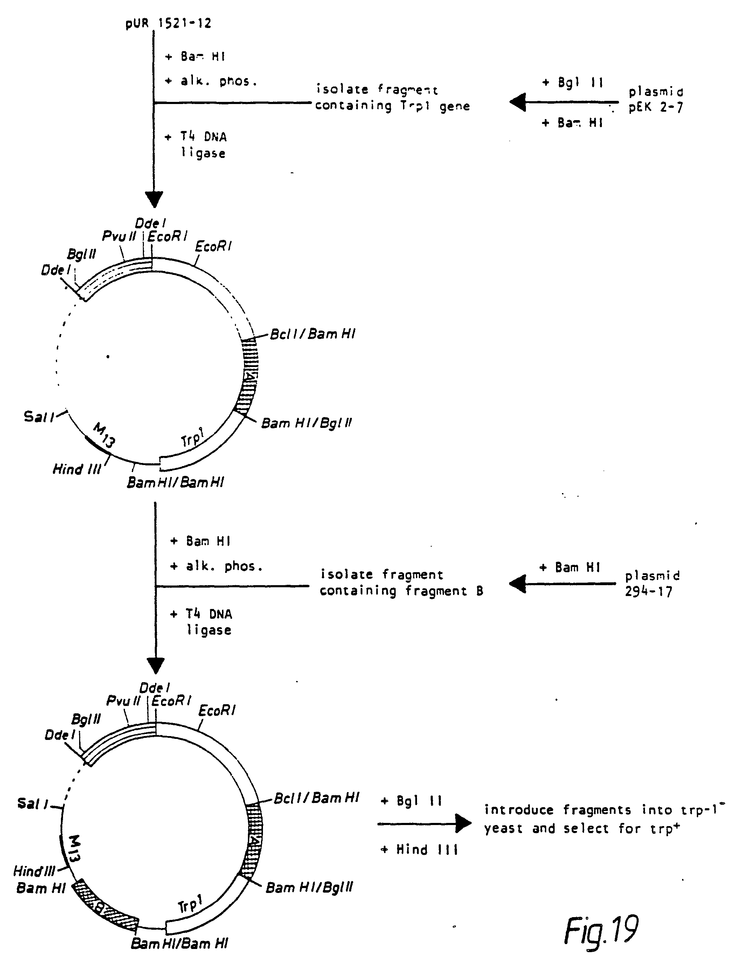

The DNA sequences according to the invention may comprise nucleotide sequences which regulate DNA replication in yeasts belonging to the genera *Saccharomyces* and *Kluyveromyces*. If the replication origin is inserted into an rDNA plasmid, the following combinations are preferred for Saccharomyces: a combination of the replication origin of the 2 micron DNA with the leu 2 gene as is present on plasmid pMP81 [C. P. Hollenberg, Current Topics in Microbiol. and Immunol. *96*, 119—144 (1982)] and a combination of the replication origin of the 2 micron DNA in combination with the trp 1 gene present on plasmid YRp7 [D. T. Stinchcomb et al., Nature *282*, 39—43 (1979)]. A preferred replication origin for *Kluyveromyces* consists of the KARS—2 sequence in combination with the trp 1 gene as is present on plasmid pEK 2—7 described in European Patent Application N° 0096430(A1) on pages 21 and 25 and in Fig. 2. If the DNA sequence has to be inserted into a yeast genome, it is preferable that the DNA sequence contains the termination region of Fig. 3 downstream of the structural gene besides the regulon region of Fig. 2 upstream of the foreign structural gene, which combination will be inserted by homologous recombination at the position of the GAPDH gene in the yeast genome (cf. Fig. 19). An alternative may be that the combination regulon region — structural gene — termination region is inserted into a cloned DNA sequence derived from yeast genome (K. Struhl, Nature *305*, 391—397 [1983] and R. J. Rothstein, Methods in Enzymology *101*, 202—211 [1983]).

For a better understanding of the invention the most important terms used in the description will be defined:

# EP 0 129 268 B1

An *operon* is a gene comprising (a) a particular DNA sequence [structural gene(s)] for polypeptide(s) expression, (b) a control region or *regulon* (regulating said expression) upstream of the structural gene and mostly consisting of a promoter regulation sequence, (c) a ribosome binding- or interaction DNA sequence and (d) a control region or transcription *terminator* downstream of the structural gene.

*Structural genes* are DNA sequences which encode through a template (mRNA) a sequence of amino acids characteristic for a specific polypeptide.

A *promoter* is a DNA sequence within the regulon to which RNA polymerase binds for the initiation of the transcription.

A *terminator* is a DNA sequence within the operon comprising amongst others particular DNA sequences involved in the polyadenylation of mRNA and particular DNA sequences involved in the termination of the transcription of DNA by RNA-polymerase.

*Reading frame.* The grouping of triplets of nucleotides (codons) into such a frame that at mRNA level a proper translation of the codons into the polypeptide takes place.

*Transcription.* The process of producing mRNA from a structural gene.

*Translation.* The process of producing a polypeptide from mRNA.

*Expression.* The process undergone by a structural gene to produce a polypeptide. It is a combination of many processes, including at least transcription and translation.

By *signal sequence* (also called signal polypeptide or leader sequence) is meant that part of the pre(pro)protein which has a high affinity to biomembranes or special receptor-proteins in biomembranes and/or which is involved in the transport/translocation of pre(pro)protein. These transport/translocation processes are often accompanied by processing of the pre(pro)protein into one of the mature forms of the protein.

*Allelic form.* One of the two or more naturally occurring alternative forms of a gene product.

*Chromosome.* Thread-like structures into which the hereditary material of cells is associated.

*Genome.* The total genetic information of cells organised in the chromosomes.

*Maturation form.* One of two or more naturally occurring forms of a gene product procured by specific processing, e.g. specific proteolysis.

By maturation forms of preprothaumatin are meant prothaumatin, prethaumatin (= preprothaumatin without a carboxylterminal sequence of 6 amino acids) and thaumatin (EP—PA 54330 and EP—PA 54331).

By maturation forms of preprochymosin are meant prochymosin, pseudo-chymosin and chymosin (EP—PA 77109).

*Plus strand.* DNA strand whose nucleotide sequence is identical with the mRNA sequence, with the proviso that uridine is replaced by thymidine.

The microbial cloning vehicles — containing (a) the various forms of the regulons and terminators (indicated with the suffix —01, —02, etc. in the plasmids described in the specification) of the glyceraldehyde-3-phosphate dehydrogenase (GAPDH) operon of *S. cerevisiae*, (b) structural genes encoding preprothaumatin and preprochymosin and their various maturation forms, (c) various hybrid forms of said structural genes encoding maturation forms of preprothaumatin or preprochymosin with special signal sequences and (d) various chemically synthesized DNA-sequences — are produced by a number of steps, the most essential of which are:

1. Isolation of clones containing the GAPDH operon of *S. cerevisiae*.

2. Isolation of the GAPDH promoter/regulation region and its introduction into plasmids encoding thaumatin-precursors.

3. Introduction of the GAPDH promoter/regulation region into plasmids encoding chymosin-precursors.

4. Reconstitution of the original GAPDH promotor/regulation region in plasmids encoding preprothaumatin by introduction of a synthetic DNA fragment (Fig. 7A, Fig. 8).

5. Reconstitution of the original GAPDH promoter/regulation region in plasmids encoding (pre)(pro)(pseudo)chymosin by introduction of a synthetic DNA fragment.

6. DNA-synthesis.

7. Structural features of the GAPDH promoter/regulation region.

8. Insertion of fragments of the GAPDH transcription termination/polyadenylation region in combination with the central transcription termination signal of phage M13RF downstream of genes encoding pseudochymosin.

9. Introduction of the 2 micron DNA replication origin and the yeast leu 2 gene in plasmids encoding thaumatin-precursors and chymosin-precursors.

10. The introduction of GAPDH transcription termination/polyadenylation regions into pURY plasmids.

11. Construction of an *E. coli*-yeast shuttle vector widely applicable for gene-expression in yeast.

12. Expression in *K. lactis* of the preprothaumatin encoding gene under control of the promoter/regulation region of the GAPDH encoding gene of *S. cerevisiae*.

13. Integration of structural genes under control of the GAPDH promoter/regulation region into the yeast chromosome.

14. Chemical synthesis of structural genes and construction of synthetically chimeric genes.

The following detailed description will illustrate the invention.

7

1. Isolation of clones containing the glyceraldehyde-3-phosphate dehydrogenase (GAPDH) operon of S. cerevisiae.

A DNA pool of the yeast S. cerevisiae was prepared in the hybrid E. coli-yeast plasmid pF1 1 [M. Chevallier et al. Gene 11, 11—10 (1980)] by a method similar to the one described by M. Carlson and D. Botstein, Cell 28, 145—154 (1982). Purified yeast DNA was partially digested with restriction endonuclease Sau 3A and the resulting DNA fragments (with an average length of 5 kb) were ligated by T4 DNA ligase in the dephosphorylated Bam HI site of pF1 1. After transformation of CaCl$_2$-treated E. coli cells with the ligated material a pool of about 30.000-ampicillin resistant clones was obtained. These clones were screened by a colony hybridization procedure [R. E. Thayer, Anal. Biochem., 98, 60—63 (1979)] with a chemically synthesized and [32]p-labelled oligomer with the sequence 5'TACCAGGAGACCAACTT3'.

According to data published by J. P. Holland and M. J. Holland [J. Biol. Chem., 255, 2596—2605, (1980)] this oligomer is complementary with the DNA sequence encoding aminoacids 306—310 (the wobble base of the last amino acid was omitted from the oligomer) of the GAPDH gene. Using hybridization conditions described by R. B. Wallace et al., Nucleic Acid Res. 9, 879—894 (1981), six positive transformants could be identified. One of these harboured plasmid pF1 1—33. The latter plasmid contained the GAPDH gene including its promoter/regulation region and its transcription termination/polyadenylation region.

The approximately 9 kb long insert of pF1 1—33 has been characterized by restriction enzyme analysis (Fig. 1) and partial nucleotide sequence analysis (Figs. 2 and 3).

Note: Unless stated otherwise, all enzyme incubations were carried out under conditions described by the supplier. Enzymes were obtained from Amersham, Boehringer, BRL or Biolabs.

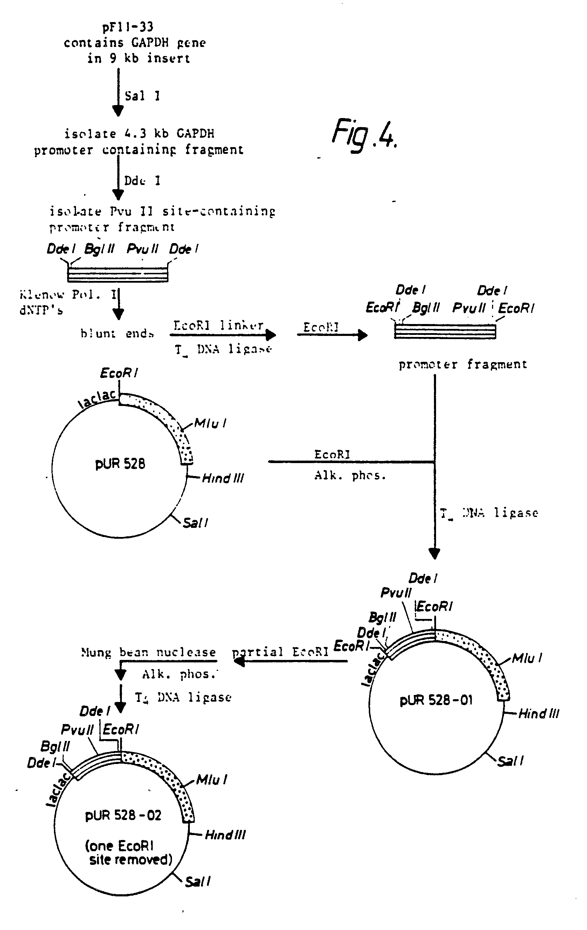

2. Isolation of the GAPDH promoter/regulation region and its introduction into plasmids encoding thaumatin-precursors (Fig. 4).

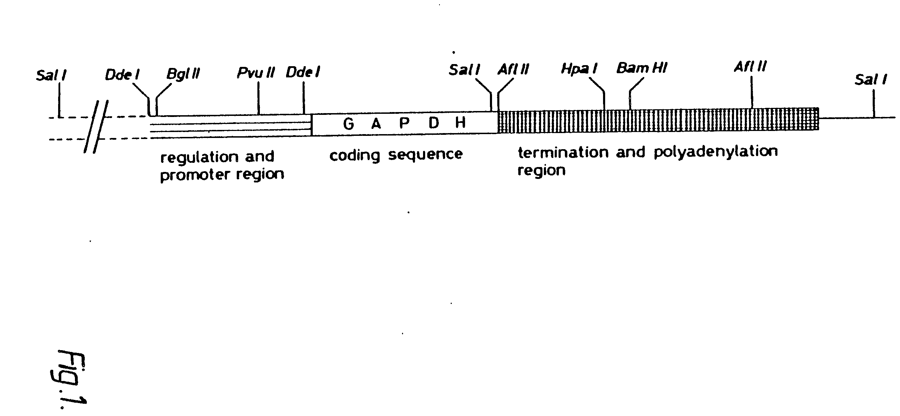

On the basis of the restriction enzyme analysis and the nucleotide sequence data of the insert of plasmid pF1 1—33, the RNA initiation regulation region of the GAPDH gene was isolated as an 800 nucleotides long Dde I fragment. To identify this promoter fragment, plasmid pF1 1—33 was digested with Sal I and the three resulting DNA fragments were subjected to a Southern hybridization test with the chemically synthesized oligomer [E. M. Southern, J. Mol. Biol. 98, 503—517 (1975)]. A positively hybridizing 4.3 kb long restriction fragment was isolated on a preparative scale by electroelution from a 0.7% agarose gel and was then cleaved with Dde I. Of the resulting Dde I fragments only the largest one had a recognition site for Pvu II, a cleavage site located within the GAPDH promoter region (Fig. 1). The largest Dde I fragment was isolated and incubated with Klenow DNA polymerase and four dNTP's (A. R. Davis et al., Gene 10, 205—218 (1980)) to generate a blunt-ended DNA molecule.

After extraction of the reaction mixture with phenol/chloroform (50/50 v/v), passage of the aqueous layer through a Sephadex G50 column and ethanol precipitation of the material present in the void volume, the DNA fragment was equipped with the $^{32}$P-labelled Eco RI linker 5'GGAATTCC3' by incubation with T4 DNA ligase. Owing to the Klenow DNA polymerase reaction and the subsequent ligation of the Eco RI linker, the original Dde I sites were reconstructed at the ends of the promoter fragment. To inactivate the ligase the reaction mixture was heated to 65°C for 10 minutes, then sodium chloride was added (final concentration 50 mmol/l) and the whole mix was incubated with Eco RI. Incubation was terminated by extraction with phenol/chloroform, the DNA was precipitated twice with ethanol, resuspended and then ligated into a suitable vector molecule. Since the Dde I promoter fragment was equipped with Eco RI sites it can be easily introduced into the Eco RI site of pUR 528, pUR 523 and pUR 522 (EP—PA 54330 and EP—PA 54331) to create plasmids in which the yeast promoter is adjacent to the structural genes encoding thaumatin precursors. The latter plasmids were obtained by cleavage of pUR 528, pUR 523 and pUR 522 with Eco RI, treatment of the linearized plasmid molecules with (calf intestinal) alkaline phosphatase to prevent self-ligation and incubation of each of these vector molecules, as well as the purified Dde I promotor fragment, with T4 DNA ligase. Transformation of the various ligation mixes in the CaCl$_2$-treated E. coli HB101 cells yielded several ampicillin resistant colonies. From some of these colonies plasmid DNA was isolated [H. C. Birnboim and J. Doly, Nucleic Acids Res. 7, 1513—1523 (1979)] and incubated with Pvu II to test the orientation of the insert.

In the nomenclature plasmids containing the Eco RI (Dde I) GAPDH promoter fragment in the correct orientation (i.e. transcription from the GAPDH promoter occurs in the direction of a downstream located structural gene) are indicated by the addendum—01 to the original code of the plasmid (for example pUR 528 is changed into pUR 528—01; see Fig. 4).

To facilitate manipulation of plasmids containing the Eco RI promoter fragment, one of the two Eco RI sites was destroyed. Two µg of plasmid DNA (e.g. pUR 528—01) was partially digested with Eco RI and then incubated with 5 units Mung bean nuclease (obtained from P. L. Biochemicals Inc.) in a total volume of 200 µl in the presence of 0.05 moles per l sodium acetate (pH 5.0), 0.05 moles/l sodium chloride and 0.001 moles/l zinc chloride for 30 minutes at room temperature to remove sticky ends. The nuclease was inactivated by addition of SDS to a final concentration of 0.1% [D. Kowalski et al., Biochemistry 15, 4457—4463 (1976)] and the DNA was precipitated by the addition of 2 volumes of ethanol (in this case the addition of 0.1 volume of 3 moles/l sodium acetate was omitted). Linearized DNA molecules were then religated by incubation with T4 DNA ligase and used to transform CaCl$_2$-treated E. coli cells. Plasmid DNA isolated from ampicillin resistant colonies was tested by cleavage with Eco RI and Mlu I for the presence of a single Eco RI site adjacent to the thaumatin gene (cf Fig. 4).

Plasmids containing the GAPDH promoter fragment, but having only a single Eco RI recognition site adjacent to the ATG initiation codon of a downstream located structural gene, are referred to as —02 type plasmids (for example: pUR 528—01 is changed into pUR 528—02; see Fig. 4).

3. Introduction of the GAPDH promoter/regulation region into plasmids encoding chymosin-precursors.

To construct plasmids containing the GAPDH promoter/regulation region adjacent to structural genes encoding chymosin precursors, use was made of plasmid pUR 528—02 (cf 2) digested with Eco RI and Hind III as a vector molecule in which various structural genes were inserted. To overcome the problem that all of the (pre)(pro)(pseudo)chymosin encoding plasmids (pUR 1524, pUR 1523 and pUR 1521, respectively, as described in EP—PA 77109) contain an additional Eco RI site within the structural gene, a Hind III digestion was carried out in combination with a partial Eco RI digest. Restriction fragments containing the intact and isolated gene were extracted from the 1% agarose gel and added to a T4 DNA ligation mix together with the vector. The vector was prepared by digesting pUR 528—02 with Hind III and Eco RI, treating the resulting fragments with phosphatase and isolation of the largest fragment from a 0.7% agarose gel. After transformation of CaCl$_2$-treated *E. coli* cells with the ligation mix and selection for ampicillin resistant colonies, plasmids containing the GAPDH promoter/regulation fragment adjacent to the (pre)(pro)(pseudo)chymosin encoding structural genes in the appropriate orientation could be isolated. In a similar way, plasmid pUR 1522 can be converted into plasmid pUR 1522—02.

Plasmids containing the GAPDH promoter fragment and the genes coding for prepro-, pro-, pseudochymosin and chymosin are referred to as pUR 1524—02, pUR 1523—02, pUR 1521—02 and pUR 1522—02, respectively.

4. Reconstitution of the original GAPDH promoter/regulation region in plasmids encoding preprothaumatin by introduction of a synthetic DNA fragment (Fig. 7A, Fig. 8).

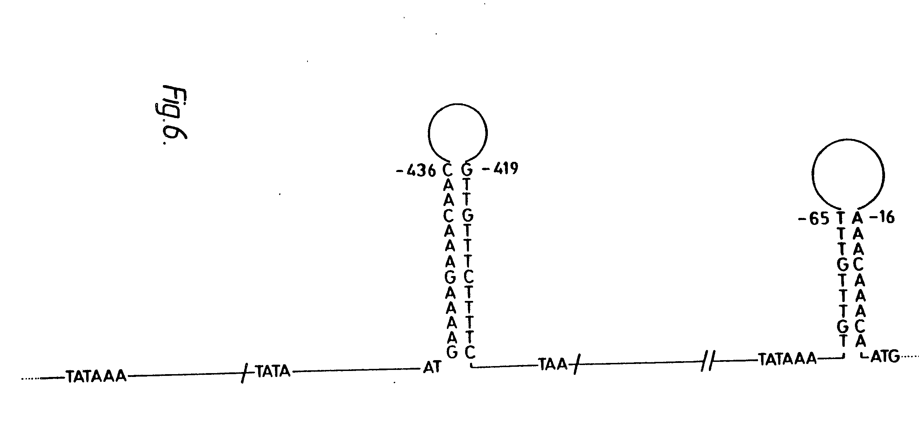

As shown by the nucleotide sequence depicted in Fig. 2, the Eco RI (Dde I) GAPDH promoter fragment contains the nucleotides —844 to —39 of the original GAPDH promoter/regulation region. Not contained in this promoter fragment are the 38 nucleotides preceding the ATG initiation codon of the GAPDH encoding gene. The latter (38) nucleotide fragment contains the PuCACACA sequence, which is found in several yeast genes. Said PuCACACA sequence located about 20 bp upstream of the translation start site [M. J. Dobson et al., Nucleic Acid Res., 10, 2625—2637 (1982)] provides the nucleotide sequence which is optimal for protein initiation [M. Kozak, Nucleic Acids Res. 9, 5233—5252 (1981)]. Moreover, as shown in Fig. 6, these 38 nucleotides allow the formation of a small loop structure which might be involved in the regulation of expression of the GAPDH gene.

On the basis of the above-mentioned arguments, introduction of the 38 nucleotides between the Dde I promoter-fragment and the ATG codon of a downstream located structural gene was considered necessary to improve promoter activity as well as translation initiation.

As outlined in Fig. 7A the missing DNA fragment was obtained by the chemical synthesis of two partially overlapping oligomers. The Sac I site present in the overlapping part of the two oligonucleotides was introduced for two reasons: (i) to enable manipulation of the nucleotide sequence immediately upstream of the ATG codon including the construction of poly A-tailed yeast expression vectors (see 11); (ii) to give a cleavage site for an enzyme generating 3'-protruding ends that can easily and reproducibly be removed by incubation with T4 DNA polymerase in the presence of the four dNTP's. Equimolar amounts of the two purified oligomers were phosphorylated at their 5'-termini, hybridized [J. J. Rossi *et al.*, (1982), J. Biol. Chem. 257, 9226—9229] and converted into a double-stranded DNA molecule by incubation with Klenow DNA polymerase and the four dNTP's under conditions which have been described for double-stranded DNA synthesis [A. R. Davis *et al.*, Gene 10, 205—218 (1980)]. Analysis of the reaction products by electrophoresis through a 13% acrylamide gel followed by autoradiography showed that more than 80% of the starting single-stranded oligonucleotides were converted into double-stranded material. The DNA was isolated by passage of the reaction mix over a Sephadex G50 column and ethanol precipitation of the material present in the void volume. The DNA was then phosphorylated by incubation with polynucleotide kinase and digested with Dde I. To remove the nucleotides cleaved off in the latter reaction, the reaction mix was subjected to two precipitations with ethanol.

As shown in Fig. 8, cloning of the resulting synthetic DNA fragment was carried out by the simultaneous ligation of this fragment and a Bgl II-Dde I GAPDH promoter regulation fragment in a vector molecule from which the Eco RI site preceding the ATG initiation codon was removed by Mung bean nuclease digestion (cf. 2). The Bgl II-Dde I promoter/regulation fragment was obtained by digestion of plasmid pUR 528—02 with Dde I and Bgl II. Separation of the resulting restriction fragments by electrophoresis through a 2% agarose gel and subsequent isolation of the fragment from the gel yielded the purified 793 nucleotides long promoter/regulation fragment. In the plasmid pUR 528—02 the nucleotide sequence preceding the ATG codon is 5'-GAATTCATG- 3' (EP—PA 54330 and EP—PA 54331), which is different from the favourable nucleotide sequence given by M. Kozak [Nucleic Acids Res. 9, 5233—5252 (1981)]. Since our aim was to reconstitute the original GAPDH promoter/regulation/protein initiation region as accurately as possible, the Eco RI site was removed in order to ligate the synthetic DNA fragment to the resulting blunt-end. Removal of the Eco RI site was accomplished by Mung bean nuclease digestion of Eco RI-cleaved pUR528—02 DNA (see 2).

Subsequently the plasmid DNA was digested with Bgl II and incubated with phosphatase. After separation of the two DNA fragments by electrophoresis through a 0.7% agarose gel, the largest fragment was isolated and used as the vector in which the Bgl II-Dde I promoter fragment as well as the —Dde I-treated- synthetic DNA fragment were ligated. Plasmids in which the Dde I promoter/regulation fragment together with the Sac I recognition site containing the synthetic DNA fragment are introduced are indicated by the addendum —03 (for example: pUR 528—02 is changed into pUR 528—03).

Similar results can be obtained with plasmids containing one of the maturation forms of preprothaumatin as the structural gene, i.e. prethaumatin, prothaumatin and thaumatin, which will result in plasmids pUR 522—03, pUR 523—03 and pUR 520—03, respectively.

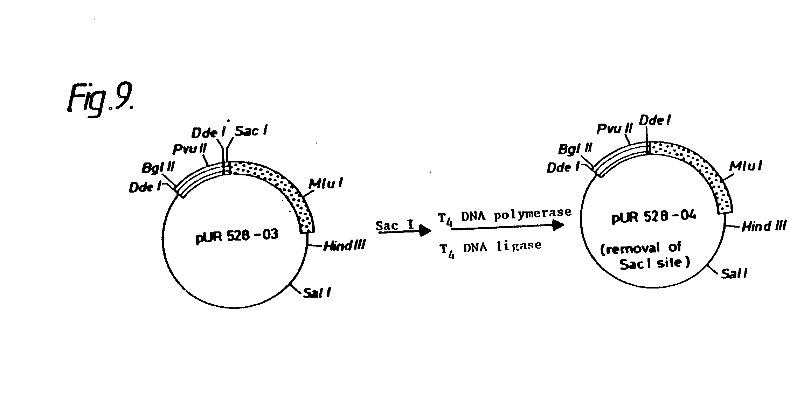

In order to reconstitute the original GAPDH promoter/regulation region as accurately as possible, the Sac I site was removed from plasmid pUR 528—03. (Fig. 9). This was accomplished by digestion of the plasmid DNA with Sac I to generate a linearized plasmid molecule with protruding 3' ends. These ends were then made blunt-ended using the 3'-exonuclease activity of T4 DNA polymerase in the presence of the four dNTP's [T. Maniatis et al. in Molecular Cloning; Cold Spring Harbor Laboratory 117—120 (1982)]. Circularisation of the linear plasmid was accomplished by using T4 DNA ligase.

Plasmids from which the Sac I site present in the synthetic DNA fragment is removed are referred to as —04 type plasmids (for example: pUR 528—03 is changed into pUR 528—04; see Fig. 9).

Similar results can be obtained with plasmids containing a structural gene for one of the maturation forms of preprothaumatin, i.e. prethaumatin, prothaumatin and thaumatin, which will result in e.g. plasmids pUR 522—04, pUR 523—04 and pUR 520—04, respectively.

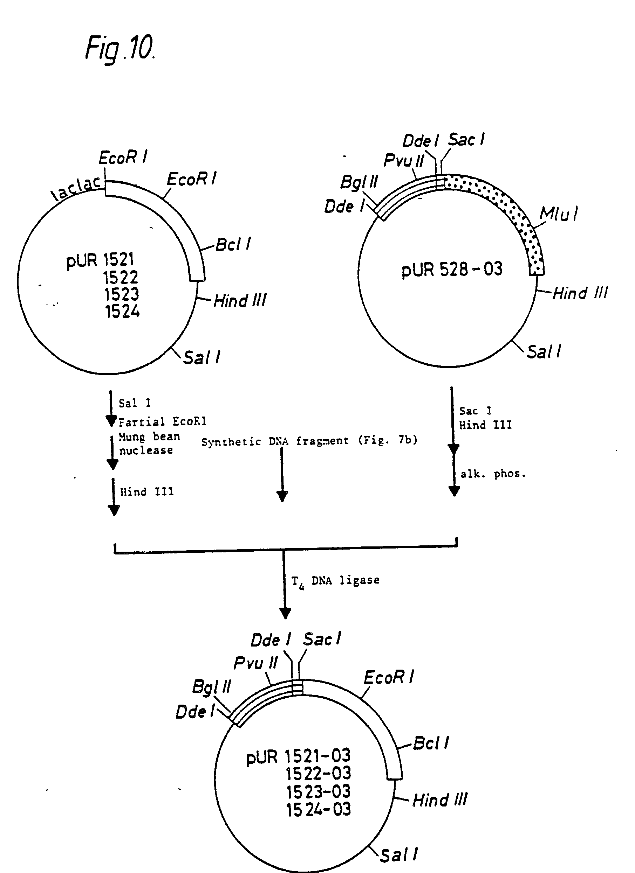

5. Reconstitution of the original GAPDH promoter/regulation region in plasmids encoding (pre)(pro)(pseudo)chymosin by introduction of a synthetic DNA fragment (Fig. 7B, Fig. 10).

To construct (pre)(pro)(pseudo)chymosin encoding plasmids containing the —03 type GAPDH promoter/regulation region (see 4), use can be made of plasmid pUR 528—03 digested with Sac I and Hind III as a vector molecule in which the various structural genes were inserted together with a synthetic DNA fragment (Fig. 10). The (pre)(pro)(pseudo)chymosin encoding genes can be isolated from the plasmids pUR 1524, pUR 1523, pUR 1521 and pUR 1522 (cf. EP PA 77109) respectively by incubation with Sal I in combination with a partial Eco RI digestion to overcome cleavage of the additional Eco RI site in the chymosin gene (cf 3). The resulting DNA fragments can then be incubated with Mung bean nuclease (cf 2) followed by a digestion with Hind III. Restriction fragments containing the intact and isolated gene can be purified by electrophoresis through a 1% agarose gel and then extracted from the gel. The synthetic DNA fragment to be used in the constructions is depicted in Fig. 7B.

The vector molecule can be prepared by digestion of plasmid pUR 528—03 with Sac I and Hind III followed by a phosphatase treatment of the restriction fragments and isolation of the largest fragment from the 0.7% agarose gel. Vector molecule, synthetic DNA fragment (Sac I-treated) and the DNA fragment containing the (pre)(pro)(pseudo)chymosin encoding nucleotide sequence can be incubated with T4 DNA ligase and transformed in $CaCl_2$-treated E. coli cells. Plasmid DNA obtained from ampicillin-resistant colonies can be tested by incubation with various restriction enzymes.

The nomenclature of the newly created plasmids is similar to the nomenclature of the (pre)(pro)-thaumatin-encoding plasmids (pUR 1524—03, pUR 1523—03, pUR 1521—03 and pUR 1522—03). Removal of the Sac I site has been described also (see 4). Removal of the Sac I sites will result in the plasmids pUR 1524—04, pUR 1523—04, pUR 1521—04 and pUR 1522—04, respectively (see Fig. 10).

6. DNA synthesis.

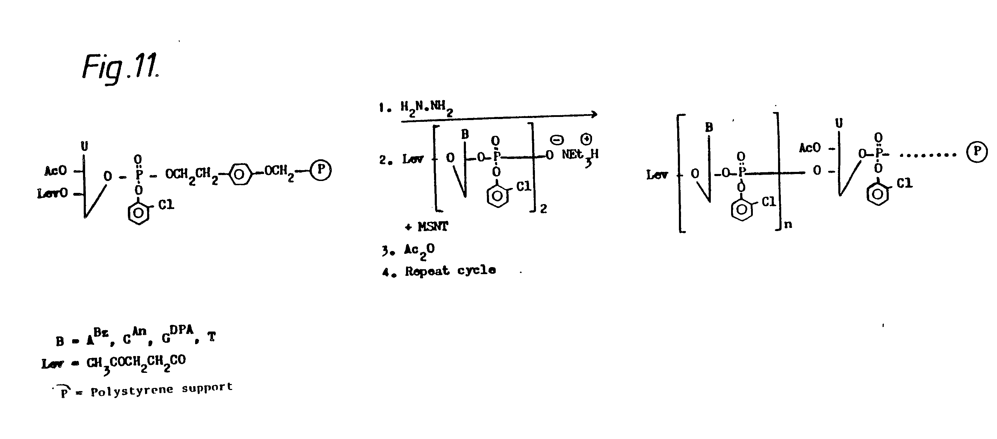

Desired oligonucleotides were synthesized on a polystyrene support using phosphotriester methodology and a library of dimers. [G. A. van der Marel et al., Recl. Trav. Chim. Pays-Bas 101, 234—241 (1982)]. Chloromethylated polystyrene (1.34 mmoles/gram) was functionalized with 2-(4-hydroxylphenyl)ethanol [Su-Sun Wang, J. Am. Chem. Soc. 95, 1325—1333 (1973)] and then coupled with 5' phosphorylated uridine protected as a mixture of 2' and 3' acetate and levulinyl groups (analogously to G. A. van der Marel et al. vide supra). This gave a support with a levulinyl functionality of 130 µmoles/g. The synthesis cycle was carried out in a 20 ml 2-necked pear shaped flask, one neck of which carried a sintered glass filter. This allowed the functionalized polystyrene (60 mg) to remain in the flask throughout the synthesis which consisted of the following steps. (cf. Fig. 11).

1). Removal of the "levulinyl" group with hydrazine hydrate (0.5 mol/l) in propionic acid/pyridine (1:3 v/v) for 5 minutes.

2). Washing with 2 × 2 ml pyridine.

3). Addition of a fourfold molar excess of the required dinucleotide anion (60 µmoles) protected in the 5' position as levulinyl ester. This was coevaporated twice with pyridine and reduced to approximately 0.5 ml before adding MSNT (240 µmoles) [C. B. Rees et al., Tetrahedron Letters 2727—2730 (1978)]. The mixture was shaken for 60 minutes (except the first cycle which was lengthened to 90 minutes).

4). Washing with 2 × 2 ml pyridine.

5). Addition of acetic anhydride (0.2 ml) and dimethyl aminopyridine in pyridine (1.5 ml, 0.05 M) to react with any unreacted hydroxyl group during 5 minutes.

6). Washing with 2 × 2 ml pyridine.

This cycle was repeated with the appropriate dimers until the desired sequence had been prepared. The 2-chlorophenyl phosphate protecting groups were removed with 0.3 mol/l 1,1,4,4-tetramethyl guanidinium 2-pyridinealdoximate (C. B. Reese et al., vide supra) in dry acetonitrile for 24 hours. After filtration and washing of the support the base protecting groups (dA = benzoyl, dC = anisoyl, dG = diphenylacetyl) were cleaved with concentrated aqueous ammonia for 60 hours at 50° which also cleaves the sequence from the uridine attached to the support [R. Crea and T. Horn, Nucleic Acids Res., 8, 2331—2348 (1980)]. Filtration of the aqueous phase from the support and evaporation gave a crude mixture which was given a clean-up by chromatography on a short column (40 cm) of Sephadex G50 with 0.05 M triethylammonium bicarbonate as eluent. Collecting and evaporating the first five fractions containing UV absorbing material gave a concentrate suitable for preparative gel electrophoresis.

7. Structural features of the GAPDH promoter/regulation region (Fig. 2, Fig. 6)

TATA or TATAAA sequences are believed to play an important role in positioning RNA initiation sites in eucaryotic promoter structures which are recognized by RNA polymerase II [C. Breathnach and P. Chambon, Annu. Rev. Biochem. 50, 349—384 (1981)]. Usually transcription is initiated 25 to 30 nucleotides 3' to such sequences although for yeast varying distances (up to 70 nucleotides; M. J. Dobson et al., Nucleic Acids Res. 10, 2625—2637 (1982)) have been described. According to the nucleotide sequence data obtained during the present investigations for the GAPDH promoter/regulation region, this structure contains two additional sets of TATA and TATAAA sequences which are located towards the ends of the promoter fragment (Fig. 2). Most likely the clustered 5'TATATAA 3' sequence occurring around position —130 is responsible for transcription of the GAPDH encoding gene. The two other TATA and TATAAA sequences present around positions —608 and —770 are possibly involved in regulation of GAPDH expression (see below; P. K. Maitra and Z. Lobo, J. Biol. Chem., 246, 489—499 (1971)). Besides these RNA initiation signals, the GAPDH promoter/regulation fragment contains various nucleotide sequences which are implicated in transcription termination. K. S. Zaret and F. Sherman [Cell 28, 563—573 (1982)] found the sequences TAG—N—TAGT—N'—TTT or TAG—N''—TATGT—N'''—TTT, in which N,N',N'' and N''' represent the variable distances between the groups, in the 3' flanking sequences of a majority of yeast genes examined. Identical or similar sequences occur in the GAPDH promoter/regulation fragment around position —625 (TAT—$N_4$—TAGT—$N_5$—TTT), around position —324 (TAC—$N_{13}$—TAGT—$N_{17}$—TTA, around position —192 (TAC—$N_9$—TATGT—$N_1$—TTT) and around position —180 (TAT—$N_{17}$—TAGT—$N_{23}$—TTT). In Fig. 6 these postulated termination signals are represented by slashes. The largest open translation reading frame present on the GAPDH promoter/regulation fragment extends from position —450 to —337 and encodes a peptide of 38 amino acids long. Since the TATA box around position —608 precedes the open reading frame it might well be that the putative peptide is translated from a transcript initiated downstream of this TATA sequence. Particularly interesting is the observation that the ATG initiation codon of the peptide forms part of a secondary structure which is generated upon base pairing of the nucleotides extending from —448 to —436 with the nucleotides extending from —419 to —407. These features are reminiscent of the situation described for the yeast leu 2 gene [A. Andreadis et al., Cell 31, 319—325 (1982)] and together with the presence of the small stem/loop structure preceding the ATG codon of the GAPDH encoding gene they might be involved in regulating the expression of the latter gene.

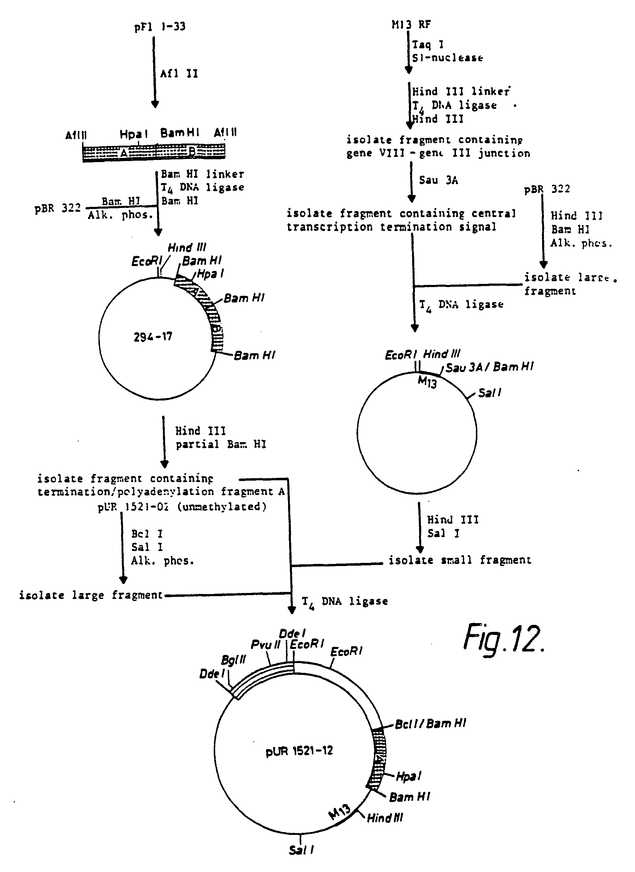

8. Insertion of fragments of the GAPDH transcription termination/polyadenylation region in combination with the central transcription termination signal of phage M13 RF downstream of genes encoding pseudochymosin (Fig. 12).

To isolate fragments of the GAPDH transcription termination/polydenylation region, plasmid pF1—33 was cleaved with restriction enzyme Afl II. Digestion with this enzyme yielded two fragments, the smaller of which has a length of 1307 nucleotides and encompasses the GAPDH termination/polyadenylation region from position 11 to 1317 (Fig. 12). The latter fragment was isolated, incubated with Mung bean nuclease to generate blunt ends (cf 2) and then equipped with the Bam HI linker (5'CCGGATCCGG3') using T4 DNA ligase. Owing to the presence of a naturally occurring Bam HI site in the middle (around position 690) of the Afl II fragment isolated, digestion of the ligation mix with Bam HI resulted in two fragments (A and B; Fig. 12). The larger of these two fragments (A) has a length of 677 nucleotides and contains the nucleotide sequence region which is located immediately downstream of the TAA translation termination codon. Attempts to subclone the purified fragment A in the Bam HI site of pBR322 proved to be unsuccessful since very few transformants were obtained and these transformants always contained fragment A as well as fragment B in various orientations. (cf. plasmid 294—17 depicted in Fig. 12).

To overcome this instability of fragment A, use was made of the central transcription termination signal of bacteriophage M13 RF [L. Edens et al., Nucleic Acids Res. 2, 1811—1820 (1975)]. M13 RF was digested with Taq I and the resulting fragments were made blunt end by incubation with Mung bean nuclease. The fragments were then equipped with a Hind III linker (5'AGAAGCTTCT3') using T4 DNA ligase followed by a digestion with Hind III. The DNA was precipitated from the reaction mix by the addition of two volumes ethanol. The precipitate was resuspended and the various restriction fragments were separated by electrophoresis through a 4% acryl-amide gel. From this gel the 441 nucleotides long fragment containing the central transcription termination signal was isolated.

After cleavage of the purified fragment with Sau 3A, the fragment containing the nucleotides 1509 to 1717 [P. M. G. van Wezenbeek et al., Gene 11, 129—148 (1980)] was closed in pBR322 which had been digested with Hind III and Bam HI (Fig. 12).

To create plasmids in which the 3'-untranslated region of the genes encoding (pre)(pro)(pseudo)chymosin was replaced by the 3'-untranslated region of the GAPDH encoding gene, plasmid pUR 1521—02 was isolated from an E. coli strain deficient in adenine methylase and cleaved with restriction endonuclease Bcl I. This enzyme recognizes the (unmethylated) sequence 5'TGATCA 3' and cleaves the (pre)(pro)(pseudo)chymosin encloding genes within their translation termination codon TGA. To generate suitable vector molecules in which both fragment A and the M13 transcription termination signal could be cloned, the Bcl I-cleaved plasmid molecules were incubated first with Sal I, followed by alkaline phosphatase. The larger of the two restriction fragments generated was isolated from the 0.7% agarose gel. For the isolation of fragment A, plasmid 294—17 was digested with Hind III and cleaved partially with Bam HI. The 1020 nucleotides long Hind III—Bam HI fragment containing the Hpa I site was obtained by electro-elution from the 1% agarose gel. The latter fragment, the vector and the 485 nucleotide long Hind III—Sal I fragment obtained from pBR322 containing the M13 transcription termination signal were incubated with T4 DNA ligase and transformed in CaCl₂-treated E. coli cells. This finally yielded the plasmid pUR 1521—12. Similar results can be obtained with plasmids pUR 1524—02, pUR 1523—02 and pUR 1522—02, which will result in plasmids pUR 1524—12, pUR 1523—12 and pUR 1522—12, respectively.

In the nomenclature plasmids containing fragment A and the M13 transcription termination signal downstream of a newly inserted structural gene are indicated by replacing the addendum —0x with —1x.

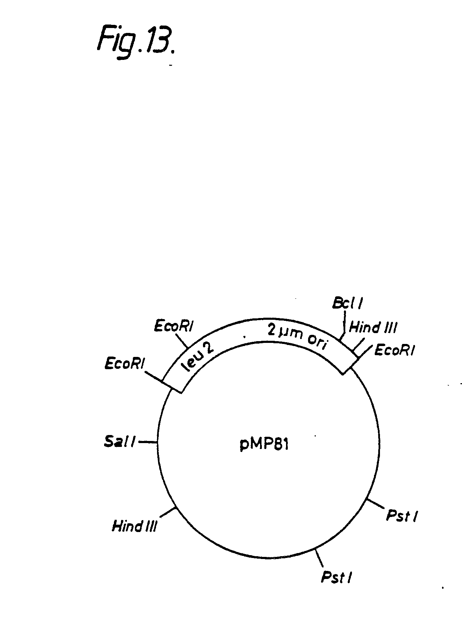

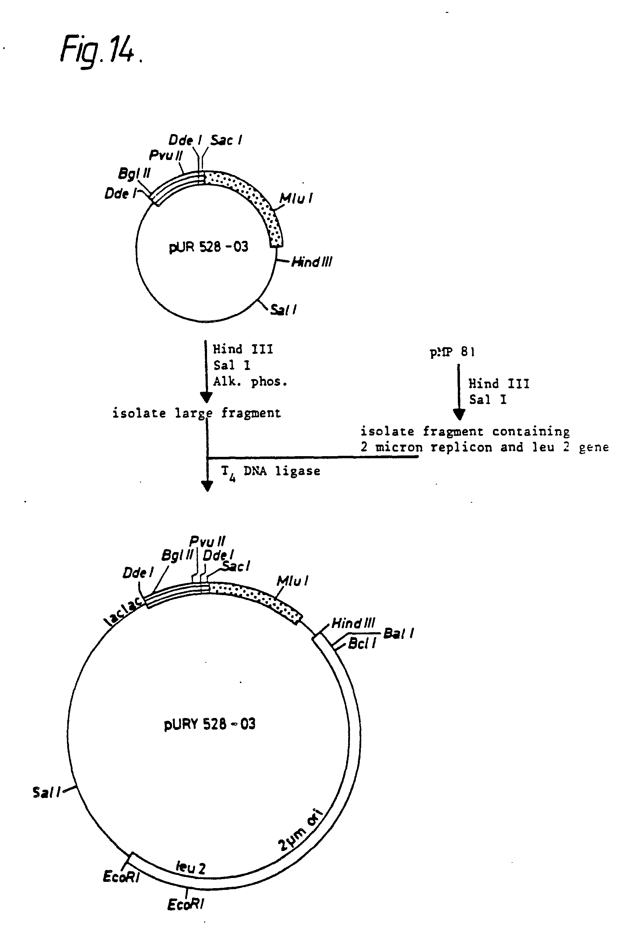

## 9. Introduction of the 2 micron DNA replication origin and the yeast leu 2 gene in plasmids encoding thaumatin-precursors and chymosin-precursors (Fig. 13, Fig. 14).

The E. coli-yeast shuttle vector pMP81 [Fig. 13; C. P. Hollenberg, Current Topics in Microbiol. and Immunol., 96, 119—144, (1982)] consists of plasmid pCRI [C. Covey et al., MGG, 145, 155—158 (1976)] and a double Eco RI fragment of pJDB 219 [J. D. Beggs, Nature, 275, 104—109 (1978)] carrying both the leu 2 gene and the yeast 2 micron DNA replication origin. The latter two functions can be excised from pMP81 by a digestion with Hind III and Sal I. The resulting 4.4 kb long restriction fragment was introduced into the various pBR 322 derivatives containing genes encoding thaumatin-precursors in combination with the various forms of the GAPDH promoter region of S. cerevisiae. A similar procedure was used to introduce the 4.4 kb long restriction fragment into the various pBR322 derivatives containing genes encoding chymosin-precursors in combination with the GAPDH promoter region of S. cerevisiae.

The introduction of the 2 micron replication origin and leu 2 gene containing Hind III—Sal I fragment into the various plasmids was accomplished by cleavage of the E. coli plasmids with Hind III and Sal I (cf. Fig. 14) and a subsequent treatment of the resulting fragments with phosphatase. After separation of the fragments by electrophoresis through a 1% agarose gel, the largest fragment was isolated, mixed with the purified Hind III—Sal I fragment obtained from pMP81, ligated with T4 DNA ligase and transformed to CaCl₂-treated E. coli cells.

In the pseudochymosin encoding plasmid containing termination fragment A (pUR 1521—12; cf. Fig. 12), the 4.4 kb long Hind III—Sal I fragment was inserted as well. For this purpose plasmid pUR 1521—12 was digested with both Hind III and Sal I (to remove the M13 transcription termination region) after which the 2 micron origin and leu 2 gene containing fragment from pMP81 cound be inserted. Unexpectedly, this plasmid construction (pURY 1521—12) proved to be stable in E. coli.

From some of the ampicillin-resistant transformants plasmid DNA was isolated and subjected to restriction enzyme analysis. Plasmids containing the correct insert were purified by CsCl-ethidium bromide density gradient centrifugation and used to transform S. cerevisiae AH22 (A. Hinnen et al., Proc. Natl. Acad. Sci. USA 75, 1929—1933 (1978)) according to the procedure of J. D. Beggs, Nature 275, 104—109 (1978). This resulted in plasmids indicated by the letter code pURY but having the same figure codes (cf. Fig. 14 in which the conversion of plasmid pUR 523—03 into pURY 528—03 is indicated). Similarly, plasmids pUR 522—02, pUR 523—02, pUR 1524—02, pUR 1523—02 and pUR 1521—02 were converted into their corresponding pURY plasmids.

With the availability of yeast transformants containing the newly constructed plasmids, the effects of plasmid variation on gene expression could be monitored. For this purpose an enzyme-linked immunosorbent assay [Elisa: A Voller et al., Bull. World Health Organ. 53, 55—65 (1976)] for the thaumatin was developed and used to quantitate the amounts of thaumatin-like protein present in yeast extracts. The results obtained in these experiments show that upon the introduction of the —02 or —03 type GAPDH promoter in pURY 528 (cf. 2, 4), thaumatin synthesis is increased by more than two orders of magnitude (more than 100 times). Upon the introduction of the 280 nucleotides long promoter fragment described by J. P. Holland and M. J. Holland [J. Biol. Chem., 255, 2596—2605, (1980)], however, thaumatin synthesis increased by order of magnitude of only one (about 10 times), hereby demonstrating the important role played by upstream sequences of the GAPDH promoter in its functioning, which is contrary to the conclusions of Holland and Holland [cf. J. Biol. Chem. 254, 9839—45 (1979)].

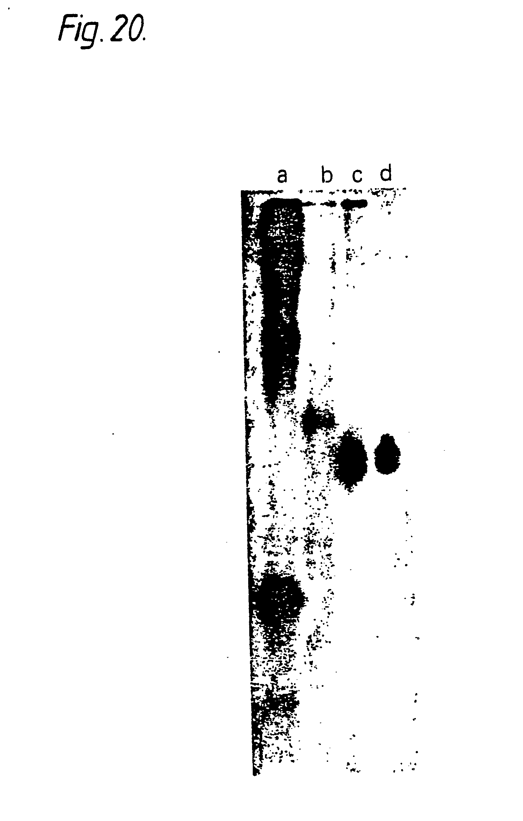

In another experiment the expression of preprothaumatin and prothaumatin encoding genes was compared. As expression of the prothaumatin encoding gene turned out to be almost negligible, this result clearly demonstrated the enormous impact which signal sequences can have on gene expression. The importance of the processing step was further substantiated by the results shown in Fig. 20. The latter experiment demonstrates that yeast cells harbouring plasmids encoding preprothaumatin are able to produce a thaumatin-like protein with a molecular weight which is practically the same as the molecular weight of thaumatin II molecules isolated from the plant.

This suggests that yeast is able to correctly process the plant signal sequence. Recently obtained data on the amino-terminal amino acid sequence of yeast-synthesized thaumatin have provided definite evidence for this notion.

In conclusion, the results obtained strongly suggest that the signal sequence plays an important role in either stimulating protein synthesis or increasing protein stability in yeasts. It is not yet sure whether prothaumatin or thaumatin is produced by yeasts in view of the smaller difference in molecular weight (about 3%) between these proteins, whereas the difference with preprothaumatin (about 10%) is easily detectable as was demonstrated in Fig. 20.

10. The introduction of GAPDH transcription termination/polyadenylation regions into pURY plasmids.

One of the possibilities to introduce GAPDH transcription termination/polyadenylation regions into pURY plasmids is to use the unique Hind III restriction site of these plasmids for insertion of various parts of the Afl II fragment depicted in Fig. 12. For example, the insertion of transcription termination fragment A (cf. Fig. 12) can be carried out as follows. Cleavage of plasmid pURY 528—03 with Hind III and a subsequent Mung bean nuclease digestion (cf. 2) will yield a linearized and blunt-ended plasmid molecule (cf. Fig 14). Incubation of this DNA with T4 DNA ligase and a suitable Bam HI linker will equip the fragment with Bam HI sites (cf. 8). Upon transformation of the Bam HI-cleaved and religated product into *E. coli*, pURY 528—03 plasmids in which the Hind III site is replaced by a Bam HI site can be recovered. Into this newly created Bam HI site transcription termination fragment A can be inserted. Digestion of the latter construction with Hpa I will indicate whether or not fragment A is inserted at the correct orientation, since in the 2 micron DNA sequence an additional Hpa I site is available.

Using the same approach but different linker molecules, different terminator fragments can be introduced at various sites in the plasmid molecule.

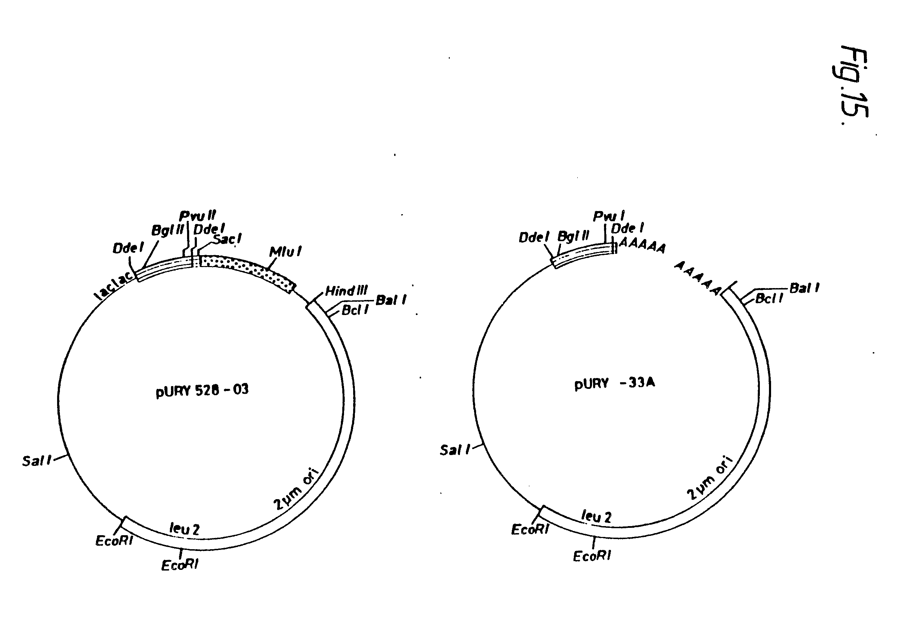

11. Construction of an *E. coli*-yeast shuttle vector widely applicable for the expression of foreign genes in yeast (Fig. 15).

Derivatives of the *E. coli*-yeast shuttle vectors described under 5 and 9, useful as generally applicable yeast expression vectors, can be prepared. In these derivatives the GAPDH promoter/regulation region including a chemically synthesized DNA fragment can be used for DNA initiation, whereas transcription termination can be effected by the transcription termination/polyadenylation region of the "Able" operon of the 2 micron DNA. Insertion of foreign genes in these expression vectors can be accomplished by homopolymer (poly dA) tailing of the Sac I site in the promoter and the Hind III site present in the "Able" operon in combination with poly dT tailing of the nucleotide sequence to be inserted.

For the preparation of the vector molecule, plasmid pURY 528—03 can be cleaved with Hind III and then incubated with Klenow DNA polymerase and the four dNTP's to generate a blunt-ended, linearized DNA molecule (Fig. 15). Subsequently this DNA molecule can be cleaved with Sac I and of the two resulting DNA fragments the larger can be isolated from a 0.7% agarose gel and incubated with terminal transferase and dATP under conditions described by G. Deng and R. Wu [Nucleic Acids Res. *9*, 4173—4188 (1981)]. The time of incubation has to be such that the tail added to the 3'end generated by cleavage with Sac I has a length of about 20 dATP residues.

The introduction of a foreign gene into the poly dA-tailed expression vector can be carried out by incubating the DNA containing this gene with a set of restriction enzymes such that the desired gene can be cleaved as close as possible upstream of the translation initiation codon and downstream of the translation termination codon of this gene. Since promoter regions can be preceded by transcription termination signals, it is important that the original promoter is not contained within the resulting DNA fragment. After purification, the trimmed DNA fragment can be equipped with poly dT tails.

If the gene to be inserted is obtained by reverse transcription of an mRNA molecule, the poly dT tails can be directly added to the S1 nuclease-treated double-stranded DNA molecule. In all cases the time of incubation with terminal transferase must be chosen such that poly dT tails with a length of about 20 nucleotides are generated. The DNA to be inserted and the poly dA-tailed vector molecule can then be hybridized by incubation at 65°C for 10 minutes, followed by cooling down the hybridization mixture slowly to room temperature. The mixture can be subsequently transformed into $CaCl_2$-treated *E. coli* cells. Plasmid DNA isolated from ampicillin-resistant transformants can then be used to transform yeast cells.

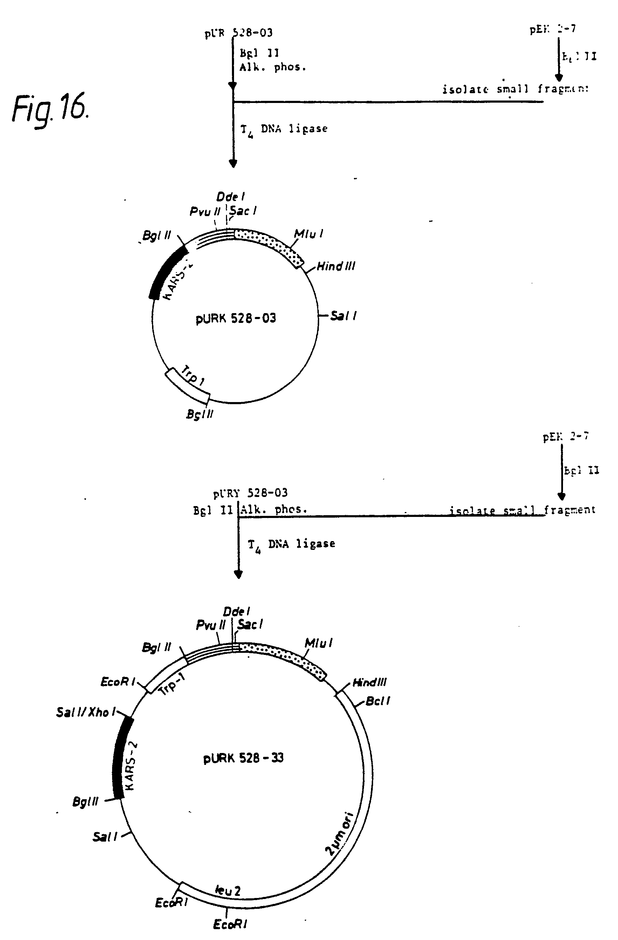

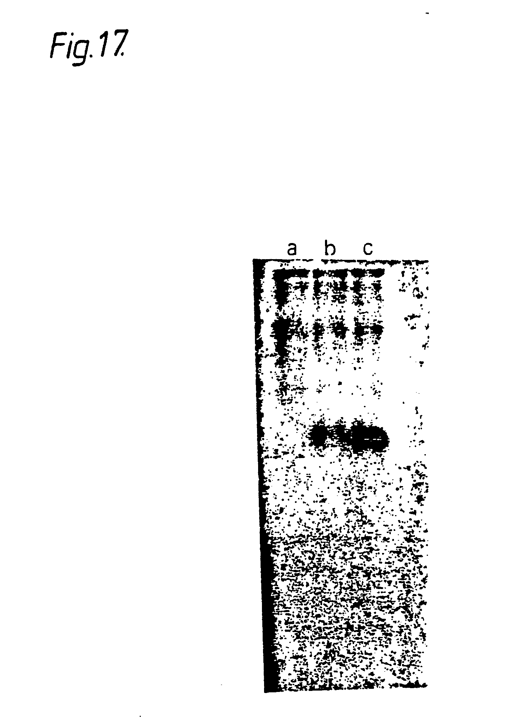

12. Expression in *K. lactis* of the preprothaumatin encoding gene under control of the promoter/regulation region of the glyceraldehyde-3-phosphate dehydrogenase encoding gene of *S. cerevisiae* (Fig. 16, Fig. 17).

The *E. coli*-yeast shuttle vector pEK 2—7 consists of plasmid YRp7 [D. T. Stinchcomb *et al.*, Nature *282*, 39—43 (1979)] containing the 1.2 kb KARS—2 fragment. Owing to the presence of the yeast trp 1 gene, plasmid pEK 2—7 can be maintained in *K. lactis* SD11 (lac 4, trp 1; cf. pages 18 and 19 of European Patent Application N° 0 096 910; A1).

To demonstrate the functionality of the promoter/regulation region of the GAPDH encoding gene in *K.*

*lactis*, plasmid pUR 528—03 (Fig. 8) has been equipped with both KARS—2 and the trp 1 gene. The latter two functions were excised from pEK 2—7 by a digestion with Bgl II followed by the isolation from a 0.7% agarose gel of the smallest fragment generated. This purified fragment was then inserted in the dephosphorylated Bgl II cleavage site of pUR 528—03 by incubation with T4 DNA ligase. Transformation of the ligation mix in CaCl$_2$-treated *E. coli* cells yielded plasmid pURK 528—03 (Fig. 16). Transformants generated by the introduction of the latter plasmid into *K. lactis* SD 11 cells by the procedure described in European Patent Application N° 0 096 910; A1 could be shown to synthesize preprothaumatin (Fig. 17).

By techniques similar to those mentioned above, plasmid pURY 528—03 was also equipped with KARS—2 and the yeast trp 1 gene and introduced into *K. lactis* SD 11 (Fig. 16). Using the same detection procedure, *K. lactis* cells carrying pURK 528—33 could also be shown to synthesize preprothaumatin (Fig. 17). Preprothaumatin production in cells containing pURK 528—33 was, however, slightly higher than in cells containing pURK 528—03. Since similar observations have been made by C. Gerbaud *et al.* [Gene 5, 233;253 (1979)] in the expression of the yeast ura 3 gene upon insertion of this gene within the coding region "Able" of the 2 micron DNA, it is very likely that the enhanced expression of preprothaumatin by pURK 528—33 is due to efficient transcription termination events in the transcription termination/polyadenylation region of the "Able" operon. This observation indicates that the presence of an efficient transcription termination/polyadenylation region downstream of a structural gene transcribed by the GAPDH promoter/regulation region is an important factor in optimizing gene expression.

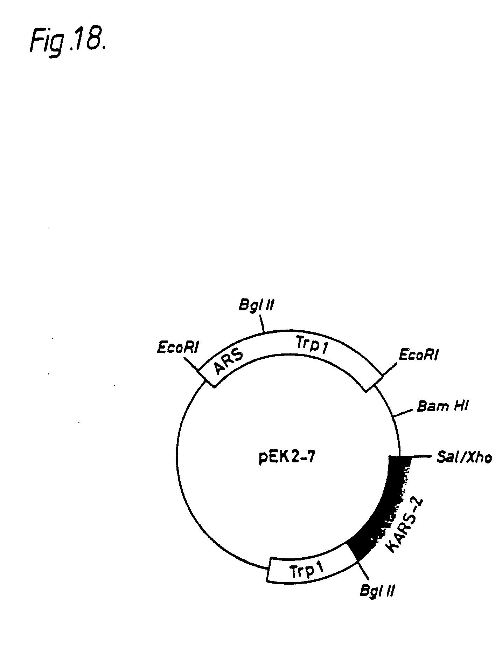

13. Integration of structural genes under the control of the GAPDH promoter/regulation region into the yeast chromosome (Fig. 18, Fig. 19).

Integration of DNA sequences encoding either a heterologous or homologous structural gene under transcriptional control of the GAPDH promoter/regulation region and, optionally, the GAPDH termination/polyadenylation region into the yeast genome, can be achieved on the basis of techniques described by R. J. Rothstein [in Methods of Enzymology *101*, 202—211 (1983)] and K. Struhl [Nature *305*, 391—397 (1983)]. The criteria to apply these techniques are the availability of (i) suitable marker genes, (ii) cloned DNA sequences homologous with DNA sequences present on the yeast genome and (iii) the availability of an intact, homologous or heterologous, protein-encoding DNA sequence.

Having the marker genes (e.g. leu 2, trp 1, his 3) and the protein-encoding DNA sequence (e.g. the sequences encoding thaumatin-precursors or chymosin-precursors) available, the latter sequences can be integrated into the yeast genome by homologous recombination events either between GAPDH promoter and terminator sequences (cf. Fig. 19) or between the marker genes. In the latter approach, which offers a variety of integration sites, the foreign protein encoding DNA sequence is integrated within the "wild type" marker gene, hereby destroying the function of this gene.

14. Chemical synthesis of structural gene.
Construction of synthetically chimeric genes.

Expression experiments of the various structural genes of preprothaumatin and preprochymosin — located on the 2 µm DNA vector and under control of the GAPDH promoter/regulation region and the GAPDH termination region — in *S. cerevisiae* resulted in an expression yield that was considered not yet economically attractive for the fermentative production of these proteins. The expression of preprothaumatin and preprochymosin genes located on the KARS-vector did not match economic feasibility either.

Another problem observed during expression experiments was that preprothaumatin was processed correctly in both *S. cerevisiae* and *K. lactis* into thaumatin (Fig. 20) (it is not clear whether the 6 amino acids on the C-terminus are also removed during processing); however, such a correct processing could not be detected with preprochymosin.

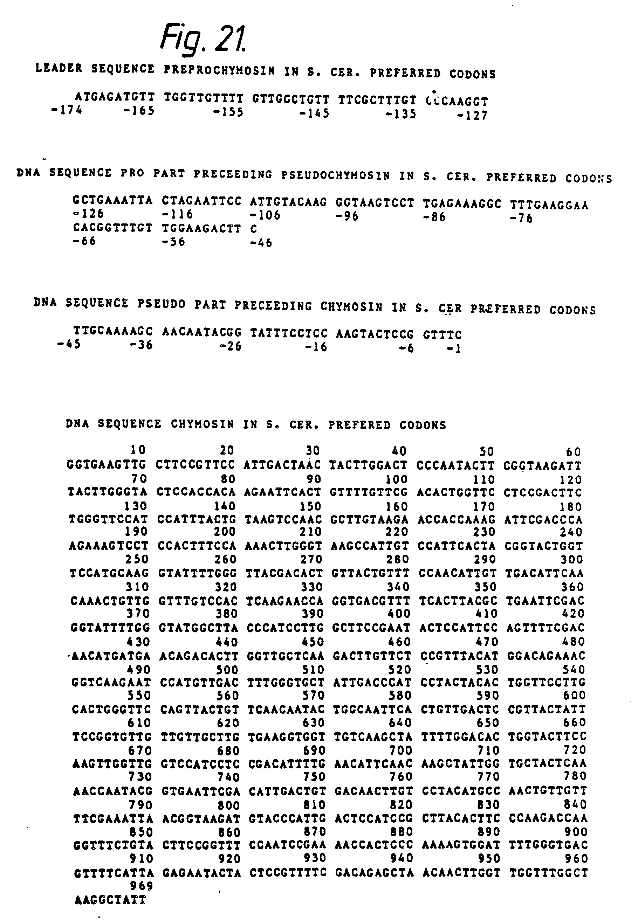

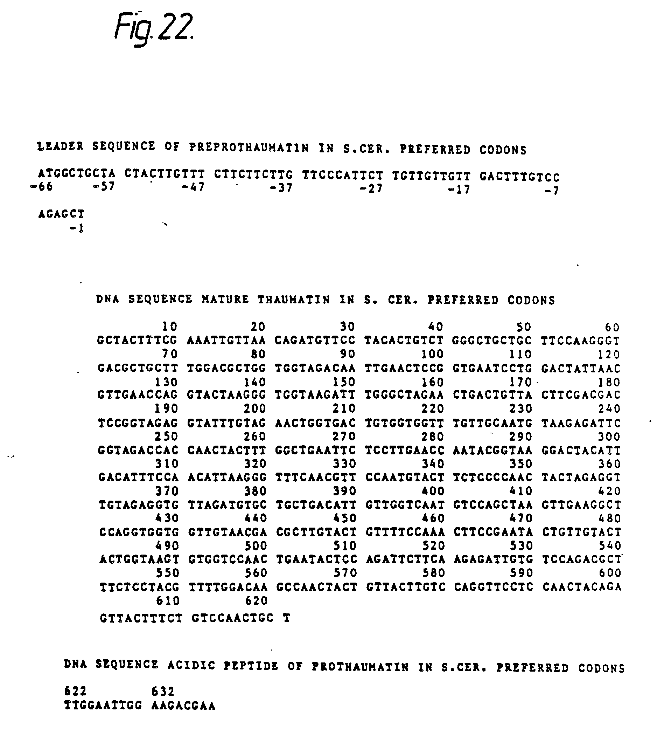

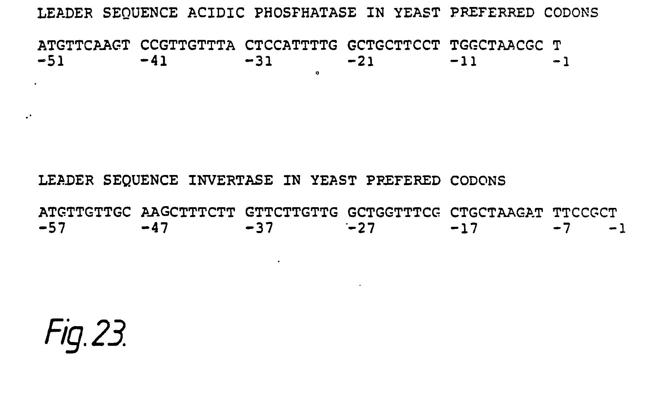

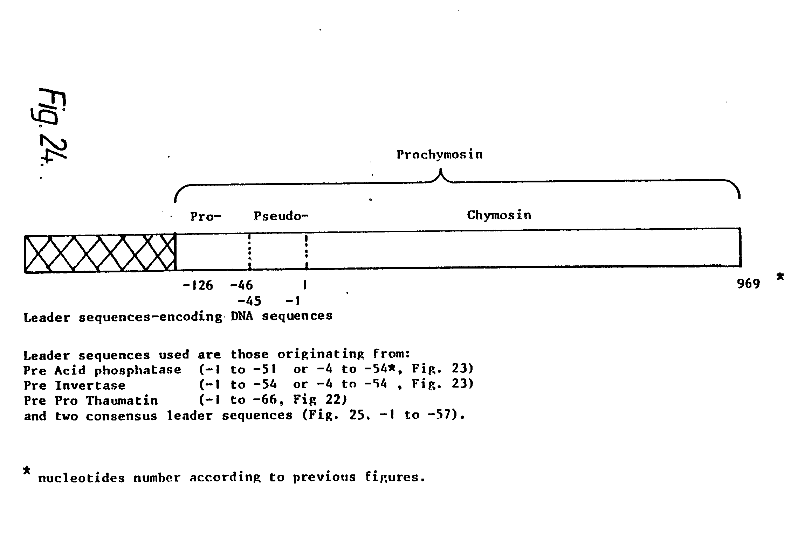

Therefore it is advocated to make preprochymosin and preprothaumatin in the preferred codons of *S. cerevisiae* [J. P. Holland and M. J. Holland; J. Biol. Chem. *255*, 2596—2605 (1980)] to increase the expression-yield. The syntheses of these genes can be carried out according to the methods described under 4 and 6. The nucleotide sequences of chymosin and thaumatin in a preferred codon usage are given in Fig. 21 and Fig. 22.

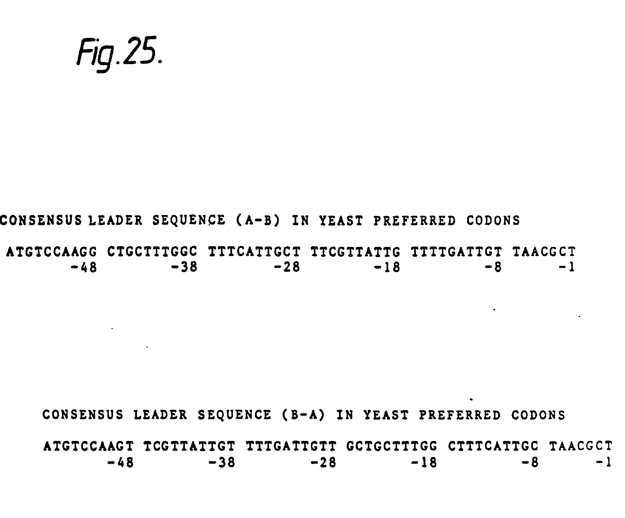

As described under 4 and 6, the synthesis can be carried out by making small (up to 30 nucleotides) single strand DNA fragments with partial overlapping sequences. This method makes it possible that also the various maturation forms of both genes are obtained simultaneously (cf. Fig. 21—22). Moreover, the approach adopted is suitable for making changes in parts of the sequences. One can use this possibility to replace the leader sequence of preprochymosin (nucleotides —174 to —127, Fig. 21) by various other leader sequences. Two typical yeast leader sequences are that of acid phosphatase [K. Arima *et al.*, Nucleic Acids Res. *11*, 1657—1672 (1983)] and that of invertase [R. Taussig and M. Carlson, Nucleic Acids Res. *11*, 1943—1954 (1983)]. Examples of nucleotide sequences encoding both leader séquences with codons preferred by yeasts are given in Fig. 23, whereas Fig. 24 gives schematically two designed chimeric acid phosphatase/prochymosin and invertase/prochymosin genes. Because preprothaumatin was processed correctly by the yeast cells, we also designed a chimeric gene of prochymosin and the leader sequence of the preprothaumatin gene (Fig. 24). Based on the physico-chemical considerations about the nature of the yeast leader sequences and the interaction of these leader sequences with the signal recognition protein [P. Walter and G. Blobel, Proc. Natl. Acad. Sci. USA, *77*, 7112—7116 (1980)], we also designed two consensus leader sequences (Fig. 25) which can be used to make chimeric genes of these consensus sequences with the prochymosin gene (Fig. 24).

Legends to the figures

Fig. 1 Restriction endonuclease cleavage map of a region of plasmid pFl 1—33 containing a yeast glyceraldehyde-3-phosphate dehydrogenase operon.

Fig. 2 Nucleotide sequence of the promoter/regulation region of a glyceraldehyde-3-phosphat dehydrogenase operon cloned in pFl 1—33. TATA and TATAAA sequences are indicated by solid underlining. Presumptive transcription termination signals are underlined with dots. Nucleotide sequences between brackets indicate inverted repeats. The nucleotide sequence encoding the 38 amino acids long peptide is enclosed in a box.

Fig. 3 Nucleotide sequence of the transcription termination/polyadenylation region of a glyceraldehyde-3-phosphate dehydrogenase operon cloned in pFl 1—33. AATAA sequences are indicated by solid underlining. Presumptive transcription termination signals are underlined with dots.

Fig. 4 Schematic representation of the insertion of the Eco Rl (Dde l) GAPDH promoter/regulation fragment in the preprothaumatin encoding plasmid and removal of an Eco Rl cleavage site from the resulting plasmid.

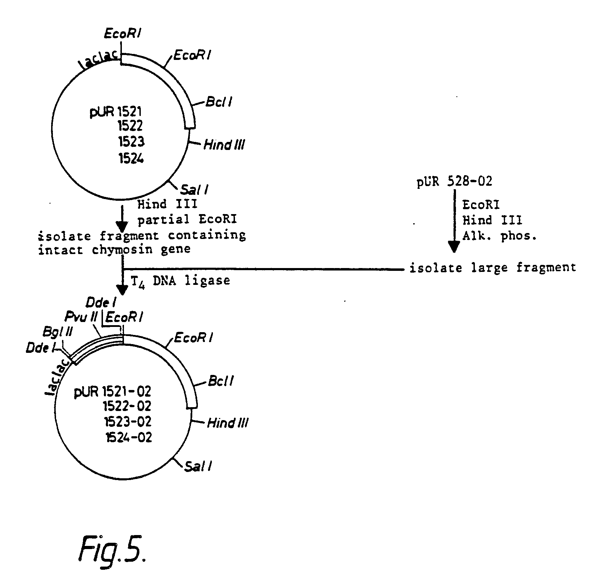

Fig. 5 Schematic representation of the construction of (pre)(pro)(pseudo)chymosin encoding plasmids containing the Eco Rl (Dde l) GAPDH promoter/regulation fragment.

Fig. 6 Schematic representation of the structure of the GAPDH promoter/regulation region including potential stem and loop structures. Presumptive transcription termination signals are indicated by slashes. The position of the coding sequence for the 38 amino acids long peptide on the fragment is shown by ATG and TAA condons.

Fig. 7a Representation of the various steps involved in the preparation of the synthetic DNA fragment used to reconstitute the original GAPH promoter/regulation region upstream of preprothaumatin encoding nucleotide sequences.

Fig. 7b Representation of the synthetic DNA fragment used to reconstitute the original GAPDH promoter/regulation region upstream of (pre)(pro)(pseudo)chymosin encoding nucleotide sequences.

Fig. 8 Schematic representation of the introduction of the synthetic DNA fragment in preprothaumatin encoding plasmids.

Fig. 9 Schematic representation of the removal of the Sac l site from the reconstituted GAPDH promoter/regulation region.

Fig. 10 Schematic representation of the introduction of the synthetic DNA fragment in (pre)(pro)(pseudo)chymosin encoding plasmids.

Fig. 11 General scheme for synthesis of DMA fragments on a polystyrene support.

Fig. 12 Schematic representation of an Afl II-Bam HI transcription termination/polyadenylation fragment obtained from pFl 1—33 and its insertion in combination with the M13 central transcription termination signal, downstream of the nucleotide sequence encoding pseudochymosin.

Fig. 13 Schematic representation of plasmid pMP81.

Fig. 14 Schematic representation of the introduction of the 2 micron DNA origin of replication and the leu 2 gene obtained from pMP81 by digestion with Hind III and Sal I into preprothaumatin encoding plasmids.

Fig. 15 Schematic representation of an E. coli-yeast shuttle vector widely applicable for the expression of foreign genes in yeast.

Fig. 16 Schematic representation of the introduction of the KARS—2 and trp 1 gene obtained from pEK 2—7 by digestion with Bg1 II into prepro-thaumatin encoding plasmids.

Fig. 17 Fluorogram of ($^{35}$S) labelled thaumatin-like proteins synthesized by K. lactis SD11 cells containing plasmid pEK 2—7 (lane a), plasmid pURK 528—03 (lane b) and plasmid pURK 528—33 (lane c) Yeast transformants were grown for 3 hours on a minimal medium containing $^{35}$S-cysteine. Cells were collected by centrifugation, resuspended in 1 ml 2.0; mol/l sorbitol, 0.025 mol/l NaPO$_4$ pH 7.5, 1 mmol/1 EDTA, 1 mmol/1 MgCL$_2$, 2.5% β-mercaptoethanol, 1 mg/ml zymolyase 60.000 and incubated for 30 minutes at 30°C. Spheroplasts were then centrifuged and lysed by the addition of 270 µl H$_2$O, 4 µl 100 mmol/1 PMSF, 8 µl 250 mmol/1 EDTA, 40 µl 9% NACl and 80 µl 5x PBSTDS (50 mmol/l NAPO$_4$ pH 7.2, 5% Triton X 100, 2.5% deoxycholate, 2,5% SDS). Immunoprecipitation of thaumatin-like proteins and analysis of precipitated proteins was carried out as described by L. Edens et al, Gene *18*, 1—12 (1982).

Fig. 18 Schematic representation of plasmid pEK 2—7.

Fig. 19 Schematic representation of a plasmid which can be used for the insertion of a pseudochymosin-encoding nucleotide sequence under transcriptional control of the GAPDH promoter/ regulation and the GAPDH termination/polyadenylation region into the yeast chromosome.

Fig. 20 Fluorogram of ($^{35}$S) cysteine labelled thaumatin-like protein synthesized by S. cerevisiae AH 22 cells containing plasmid pURY 528—03 (lane c) or sythesized *in vitro* in a wheat germ protein synthesizing system under the direction of mRNA purified from arils of Thaumatococcus fruits (lane b). Lane a shows radioactive marker proteins obtained from Amersham) and lane d shows the position of thaumatin II isolated from the arils of Thaumatococcus fruits. Lysis of yeast cells was carried out as described in the legend of fig. 17. Wheat germ translation of mRNA and immunoprepipitation procedures were carried out as described by L. Edens et al, Gene 18, 1—12 (1982).

Fig. 21 Nucleotide sequence of the gene encoding (pre)(pro)(pseudo)chymosin in codon usage preferred by S. cerevisiae.

Fig. 22 Nucleotide sequence of the gene encoding (pre)(pro)thaumatin in codon usage preferred by S. cerevisiae.

Fig. 23 Nucleotide sequence of the genes encoding the leader sequences acid phosphatase and invertase in codon usage preferred by S. cerevisiae.

Fig. 24 Schematic representation of the construction of the gene encoding prochymosin provided with the leader sequences of the invertase, acid phosphatase or thaumatin encoding genes, or with the two designed consensus leader sequences.

Fig. 25 Nucleotide sequences of the two designed consensus leader sequences.

## Claims for the Contracting States: BE CH DE FR GB IT LI NL SE

1. A DNA sequence giving an improved expression of newly introduced genes in yeast cells, comprising a DNA sequence capable of initiating transcription by yeast RNA polymerase II, which latter DNA sequence is derived from the regulon region of one of the GAPDH genes of S. cerevisiae and essentially consists of polynucleotide −850 to −1 of Figure 2, namely

```
-850         -840         -830         -820         -810         -800
GAATTCCTCA   GTTTCAAGAT   CTTTTAATGT   CCAAAACCAT   TTGAGCCGAT   CTAAATACTT
-790         -780         -770         -760         -750         -740
CTGTGTTTTC   ATTAATTTAT   AAATTGTACT   CTTTTAAGAC   ATGGAAAGTA   CCAACATCGG
-730         -720         -710         -700         -690         -680
TTGAAACAGT   TTTTCATTTA   CATATGGTTT   ATTGGTTTTT   CCAGTGAATG   ATTATTTGTC
-670         -660         -650         -640         -630         -620
GTTACCCTTT   CGTAAAACTT   CAAACACGTT   TTTAAGTATT   GTTTAGTTGC   TCTTTCGACA
-610         -600         -590         -580         -570         -560
TATATGATTA   TCCCTGCGCG   GCTAAAGTTA   AAGATGCAAA   AAACAGAAGA   CAACTGAAGT
-550         -540         -530         -520         -510         -500
TAATTTACGT   CAATTAAGTT   TTCCAGGCTA   ATGATGTTTT   GGGCTTCCAC   TAATTCAATA
-490         -480         -470         -460         -450         -440
AGTATGTCAT   GAAATACGTT   GTGAAGAGCA   TCCAGAAATA   ATGAAAAGAA   ACAACGAAAC
-430         -420         -410         -400         -390         -380
TGGGTCGGCC   TGTTGTTTCT   TTTCTTTACC   ACGTGATCTG   CGGCATTTAC   AGGAAGTCGC
-370         -360         -350         -340         -330         -320
GCGTTTTGCG   CAGTTGTTGC   AACGCAGCTA   CGGCTAACAA   AGCCTAGTGG   AACTCGACTG
-310         -300         -290         -280         -270         -260
ATGTGTTAGG   GCCTAAAACT   GGTGGTGACA   GCTGAAGTGA   ACTATTCAAT   CCAATCATGT
-250         -240         -230         -220         -210         -200
CATGGCTGTC   ACAAAGACCT   TGCGGACCGC   ACGTACGAAC   ACATACGTAT   GCTAATATGT
-190         -180         -170         -160         -150         -140
GTTTTGATAG   TACCCAGTGA   TCGCAGACCT   GCAATTTTTT   TGTAGCTTTG   GAAGAATATA
-130         -120         -110         -100          -90          -80
TAAAGGTTGC   ACTCATTCAA   GATAGTTTTT   TTCTTGTGTG   TCTATTCATT   TTATTATTGT
 -70          -60          -50          -40          -30          -20
TTGTTTAAAT   GTTAAAAAAA   CCAAGAACTT   AGTTTCAAAT   TAAATTCATC   ACACAAACAA
 -10          -1
ACAAAACAAA
```

wherin the regulon is optionally modified to include at least one restriction enzyme cleavage site, to facilitate manipulation of the nucleotide sequence region for protein synthesis initiation.

2. DNA sequence, which can be inserted into a recombinant DNA plasmid or into a yeast chromosome, comprising:

(a) a DNA sequence according to claim 1, capable of initiating transcription by yeast RNA polymerase II, and

(b) one or more structural genes different from the GAPDH genes of S. cerevisial, and at least two of features (c)-(f),

(c) one or more specific DNA sequences capable of terminating the transcription by yeast RNA polymerase II and effecting polyadenylation of the mRNA, and/or

(d) one or more selection markers, and/or

(e) either one or more nucleotide sequences allowing a stable insertion in a chromosome of yeasts or one or more DNA sequences which regulate replication in yeasts belonging to the genus Saccharomyces or to the genus *Kluyveromyces*, and/or

(f) a DNA sequence encoding a signal polypeptide of not more than 30 amino acid residues assisting the translocation of proteins, which DNA sequence is situated upstream of and in the same reading frame as the structural gene.

3. DNA sequence according to claim 2, characterized in that the structural gene of (b) encodes thaumatic or chymosin, or their various allelic or modified forms, which modified forms do not impair the sweet-tasting properties of thaumatin or the milk-clotting properties of chymosin, respectively, or precursors of these thaumatin-like or chymosin-like proteins.

4. DNA sequence according to claim 2, characterized in that the sepcific DNA sequence of (c) is a DNA sequence capable of both termination of the transcription by yeast RNA polymerase II and effecting polyadenylation of the mRNA, which DNA sequence is derived from the termination/polyadenylation region belonging to one of the GAPDH genes is *S. cerevisial* and comprises at least the *Hpa* I site-containing *Afl* II-*Bam* HI fragment A essentially having the formula of polynucleotide 15—687 of Figure 3, namely

```
                        17         27         37         47         57
                  GCT TACTTTAATG ATTTACTTTT TATTATTAAT AATTCATGCT
        67         77         87         97        107        117
CATGACATCT CATATACACG TTTATAAAAC TTAAATAGAT TGAAAATGTA TTAAAGATTC
       127        137        147        157        167        177
CTCAGGGATT CGATTTTTTT GGAAGTTTTT GTTTTTTTTT CCTTGAGATG CTGTACTATT
       187        197        207        217        227        237
TGGGAACAAT TATACAATCG AAAGATATAT GCTTACATTC GACCGTTTTA GCCGTGATCA
       247        257        267        277        287        297
TTATCCTATA GTAACATAAC CTGAACTATA ACTGACACTA CTATCATCAA TACTTGTCAC
       307        317        327        337        347        357
ATGAGAACTC TCTGAATAAT TACGCCACTG AAATTTGATG CCTGAACGAC CGGCATCACG
       367        377        387        397        407        417
TATCTTCGAT AAAGCACTTA GTATCACACT AATTGGCTTT TCGCCGCATA TGGTGTTTCC
       427        437        447        457        467        477
GGTGATTTCC AAGTATTGTT TCCAAGCATC GTACCTTTCA CCATTTGGAG TATCACTTAG
       487        497        507        517        527        537
CGTTTTCATC GCATATCTGT CCATTATTTC AATGGATTGC CAAATGGGAA CTTGATGATG
       547        557        567        577        587        597
TGAAAGTTTA CTCCTAGCAG TTAACATTTC CACTTCTGTT TCCTCTTTAA TGGCATTCAT
       607        617        627        637        647        657
TCAACTCTTC CTTGCTTACC GACGTACCCG TATATTGGAA TCTGCGGCCC CAATGACAGA
       667        677        687
AATCACTGCT TACAATGAAT AAATTGTTCG.
```

5. DNA sequence according to claim 2, characterized in that the DNA sequence of (f) encoding a signal polypeptide is selected from the group consisting of DNA sequences encoding

(a) the signal polypeptide translocating *S. cerevisial* invertase, namely Met.Leu.Leu.Gln.Ala.Phe.Leu.Phe.Leu.Leu.Ala.Gly.Phe.Ala.Ala.Lys.Ile.Ser.Ala;

(b) the signal polypeptide translocating *S. cerevisial* acid phosphatase, namely Met.Phe.Lys.Ser.Val.Val.Tyr.Ser.Ile.Leu.Ala.Ala.Ser.Leu.Ala.Asn.Ala;

(c) the signal polypeptide of unmatured forms of thaumatin-like proteins, namely Met.Ala.Ala.Thr.Thr.Cys.Phe.Phe.Phe.Leu.Phe.Pro.Phe.Leu.Leu.Leu.Leu.Thr.Leu.Ser.Arg.Ala;

(d) the signal polypeptide of unmatured forms of chymosin-like proteins, namely Met.Arg.Cys.Leu.Val.Val.Leu.Leu.Ala.Val.Phe.Ala.Leu.Ser.Gln.Gly; and

(e) two consensus signal polypeptides, namely
Met.Ser.Lys.Ala.Ala.Leu.Ala.Phe.Ile.Ala.Phe.Val.Ile.Val.Leu.Ile.Val.Asn.Ala and
Met.Ser.Lys.Phe.Val.Ile.Val.Leu.Ile.Val.Ala.Ala.Leu.Ala.Phe.Ile.Ala.Asn.Ala.

6. DNA sequence according to claim 2, characterized in that either the codons of the structural gene of (b) or the codons of the signal polypeptide-encoding DNA sequence of (f), or both, are modified into codons preferred by yeasts.

7. DNA sequence according to claim 6, characterized in that as codons preferred by yeasts the following codons are used:

| codon | encoding | codon | encoding |
|---|---|---|---|
| GCC or GCT | alanine | TTG | leucine |
| AGA | arginine | AAG | lysine |
| AAC | asparagine | ATG | methionine |
| GAC or GAT | aspartic acid | TTC | phenylalanine |
| TGT | cysteine | CCA | proline |
| CAA | glutamine | TCC or TCT | serine |
| GAA | glutamic acid | ACC or ACT | threonine |
| GGT | glycine | TGG | tryptophan |
| CAC | histidine | TAC | tyrosine |
| ATC or ATT | isoleucine | GTC or GTT | valine. |

8. Process for preparing yeasts containing rDNA sequences, characterized in that DNA sequences as claimed in claim 2 are introduced into *Saccharomyces*, *Kluyveromyces*, *Hansenula*, *Candida* or *Torulopsis*, yeasts, either in the form of plasmids or by incorporation in the yeast genome.

9. Yeast containing an rDNA sequence as claimed in claim 2, either in the form of a plasmid or incorporated in the yeast genome.

10. Process for preparing a protein by cultivation of a yeast containing rDNA sequences, characterized in that a yeast as claimed in claim 9 is used to produce a protein, whereby the rDNA sequence contains a structural gene encoding that protein or a precursor thereof which precursor during processing can form the relevant protein.

11. Process according to claim 10, in which the protein is a thaumatin-like protein.

12. Process according to claim 10, in which the protein is a chymosin-like protein.

**Claims for the Contracting State: AT**

1. Process for preparing a yeast containing an rDNA sequence, characterized in that a DNA sequence, which can be inserted into a recombinant DNA plasmid or into a yeast chromosome, comprising:

(a) a DNA sequence giving an improved expression of newly introduced genes in yeast cells, comprising a DNA sequence capable of initiating transcription by yeast RNA polymerase II, which latter DNA sequence is derived from the regulon region of one of the GAPDH genes of *S. cerevisiae* and essentially consists of polynucleotide.−850 to −1 of Figure 2, namely

```
-850        -840        -830        -820        -810        -800
GAATTCCTCA  GTTTCAAGAT  CTTTTAATGT  CCAAAACCAT  TTGAGCCGAT  CTAAATACTT
-790        -780        -770        -760        -750        -740
CTGTGTTTTC  ATTAATTTAT  AAATTGTACT  CTTTTAAGAC  ATGGAAAGTA  CCAACATCGG
-730        -720        -710        -700        -690        -680
TTGAAACAGT  TTTTCATTTA  CATATGGTTT  ATTGGTTTTT  CCAGTGAATG  ATTATTTGTC
-670        -660        -650        -640        -630        -620
GTTACCCTTT  CGTAAAACTT  CAAACACGTT  TTTAAGTATT  GTTTAGTTGC  TCTTTCGACA
-610        -600        -590        -580        -570        -560
TATATGATTA  TCCCTGCGCG  GCTAAAGTTA  AAGATGCAAA  AAACAGAAGA  CAACTGAAGT
-550        -540        -530        -520        -510        -500
TAATTTACGT  CAATTAAGTT  TTCCAGGGTA  ATGATGTTTT  GGGCTTCCAC  TAATTCAATA
-490        -480        -470        -460        -450        -440
AGTATGTCAT  GAAATACGTT  GTGAAGAGCA  TCCAGAAATA  ATGAAAAGAA  ACAACGAAAC
-430        -420        -410        -400        -390        -380
TGGGTCGGCC  TGTTGTTTCT  TTTCTTTACC  ACGTGATCTG  CGGCATTTAC  AGGAAGTCGC
-370        -360        -350        -340        -330        -320
GCGTTTTGCG  CAGTTGTTGC  AACGCAGCTA  CGGCTAACAA  AGCCTAGTGG  AACTCGACTG
-310        -300        -290        -280        -270        -260
ATGTGTTAGG  GCCTAAAACT  GGTGGTGACA  GCTGAAGTGA  ACTATTCAAT  CCAATCATGT
-250        -240        -230        -220        -210        -200
CATGGCTGTC  ACAAAGACCT  TGCGGACCGC  ACGTACGAAC  ACATACGTAT  GCTAATATGT
-190        -180        -170        -160        -150        -140
GTTTTGATAG  TACCCAGTGA  TCGCAGACCT  GCAATTTTTT  TGTAGGTTTG  GAAGAATATA
-130        -120        -110        -100        -90         -80
TAAAGGTTGC  ACTCATTCAA  GATAGTTTTT  TTCTTGTGTG  TCTATTCATT  TTATTATTGT
-70         -60         -50         -40         -30         -20
TTGTTTAAAT  GTTAAAAAAA  CCAAGAACTT  AGTTTCAAAT  TAAATTCATC  ACAGAAACAA
-10         -1
ACAGAAACAAA
```

wherein the regulon is optionally modified to include at least one restriction enzyme cleavage site, to facilitate manipulation of the nucleotide sequence region for protein synthesis initiation, and

(b) one or more structural genes different from the GAPDH genes of *S. cerevisial*, and at least two of features (c)-(f),

(c) one or more specific DNA sequences capable of terminating the transcription by yeast RNA polymerase II and effecting polyadenylation of the mRNA, and/or

(d) one or more selection markers, and/or

(e) either one or more nucleotide sequences allowing a stable insertion in a chromosome of yeasts or one or more DNA sequences which regulate replication in yeast belonging to the genus *Saccharomyces* or to the genus *Kluyveromyces*, and/or

(f) a DNA sequence encoding a signal polypeptide of not more than 30 amino acid residues assisting the translocation of proteins, which DNA sequence is situated upstream of and in the same reading frame as the structural gene is introduced into *Saccharomyces*, *Kluyveromyces*, *Hansenula*, *Candida*, or *Torulopsis* yeast, either in the form of a plasmid or by incorporation in the yeast genome.

2. Process according to claim 1, characterized in that the structural gene of (b) encodes thaumatin or chymosin, or their various allelic or modified forms, which modified forms do not impair the sweet-tasting properties of thaumatin or the milk-clotting properties of chymosin, respectively, or precursors of these thaumatin-like or chymosin-like proteins.

3. Process according to claim 1, characterized in that the specific DNA sequence of (c) is a DNA sequence capable of both termination of the transcription by yeast RNA polymerase II and effecting polyadenylation of the mRNA, which DNA sequence is derived from the termination/polyadenylation region belonging to one of the GAPDH genes is *S. cerevisial* and comprises at least the *Hpa* I site-containing *Afl* II-*BAm* HI fragment A essentially having the formula of polynucleotide 15—687 of Figure 3, namely

```
                        17          27          37         .47          57
                        GGT  TACTTTAATG  ATTTACTTTT  TATTATTAAT AATTCATGCT
             67          77          87          97         107         117
   CATGACATCT CATATACACG TTTATAAAAC TTAAATAGAT TGAAAATGTA TTAAAGATTC
            127         137         147         157         167         177
   CTCAGGGATT CGATTTTTTT GGAAGTTTTT GTTTTTTTTT CCTTGACATG CTGTAGTATT
            187         197         207         217         227         237
   TGGGAACAAT TATACAATCG AAAGATATAT GCTTACATTC GACCGTTTTA GCCGTGATCA
            247         257         267         277         287         297
   TTATCCTATA GTAACATAAC CTGAAGTATA ACTGACACTA CTATCATCAA TACTTGTCAC
            307         317         327         337         347         357
   ATGAGAACTC TGTGAATAAT TAGGCCACTG AAATTTGATG CCTGAAGGAC CGGCATCACG
            367         377         387         397         407         417
   TATCTTCGAT AAAGCACTTA GTATCACACT AATTGGCTTT TCGCCGCATA TGGTGTTTCC
            427         437         447         457         467         477
   GGTGATTTCC AAGTATTGTT TCCAAGCATC GTACCTTTCA CCATTTGGAG TATCACTTAG
            487         497         507         517         527         537
   CGTTTTCATC GCATATCTGT CCATTATTTC AATGGATTGC CAAATGGGAA CTTGATGATG
            547         557         567         577         587         597
   TGAAAGTTTA CTCCTAGCAG TTAACATTTC CACTTCTGTT TCCTCTTTAA TGGCATTCAT
            607         617         627         637         647         657
   TCAACTCTTC CTTGCTTACC GACGTACCCG TATATTGGAA TCTGCGGCCC CAATGACAGA
            667         677         687
   AATCACTGCT TACAATGAAT AAATTGTTCG.
```

4. Process according to claim 1, characterized in that the DNA sequence of (f) encoding a signal polypeptide is selected from the group consisting of DNA sequences encoding

(a) the signal polypeptide translocating *S. cerevisial* invertase, namely Met.Leu.Leu.Gln.Ala.Phe.Leu.Phe.Leu.Leu.Ala.Gly.Phe.Ala.Ala.Lys.Ile.Ser.Ala;

(b) the signal polypeptide translocating *S. cerevisial* acid phosphatase, namely Met.Phe.Lys.Ser.Val.Val.Tyr.Ser.Ile.Leu.Ala.Ala.Ser.Leu.Ala.Asn.Ala;

(c) the signal polypeptide of unmatured forms of thaumatin-like proteins, namely Met.Ala.Ala.Thr.Thr.Cys.Phe.Phe.Phe.Leu.Phe.Pro.Phe.Leu.Leu.Leu.Leu.Thr.Leu.Ser.Arg.Ala;

(d) the signal polypeptide of unmatured forms of chymosin-like proteins, namely Met.Arg.Cys.Leu.Val.Val.Leu.Leu.Ala.Val.Phe.Ala.Leu.Ser.Gln.Gly; and

(e) two consensus signal polypeptides, namely
Met.Ser.Lys.Ala.Ala.Leu.Ala.Phe.Ile.Ala.Phe.Val.Ile.Val.Leu.Ile.Val.Asn.Ala and
Met.Ser.Lys.Phe.Val.Ile.Val.Leu.Ile.Val.Ala.Ala.Leu.Ala.Phe.Ile.Ala.Asn.Ala.

5. Process according to claim 1, characterized in that either the codons of the structural gene of (b) or the codons of the signal polypeptide-encoding DNA sequence of (f), or both, are modified into codons preferred by yeasts.

6. Process according to claim 1, characterized in that as codons preferred by yeasts the following codons are used:

| codon | encoding | codon | encoding |
|---|---|---|---|
| GCC or GCT | alanine | TTG | leucine |
| AGA | arginine | AAG | lysine |
| AAC | asparagine | ATG | methionine |
| GAC or GAT | aspartic acid | TTC | phenylalanine |
| TGT | cysteine | CCA | proline |
| CAA | glutamine | TCC or TCT | serine |
| GAA | glutamic acid | ACC or ACT | threonine |
| GGT | glycine | TGG | tryptophan |
| CAC | histidine | TAC | tyrosine |
| ATC or ATT | isoleucine | GTC or GTT | valine. |

7. Process for preparing a protein by cultivation of a yeast containing rDNA sequences, characterized in that a yeast prepared by a process as claimed in any of claims 1—6, or its progeny, is used to produce a protein, whereby the rDNA sequence contains a structural gene encoding that protein or a precursor thereof which precursor during processing can form the relevant protein.

8. Process according to claim 7, in which the protein is a thaumatin-like protein.

9. Process according to claim 7, in which the protein is a chymosin-like protein.

**Patentansprüche für die Vertragsstaaten: BE CH LI DE FR IT NL SE GB**

1. DNA-Sequenz zur Erzielung einer verbesserten Expression neu eingeführter Gene in Hefezellen, umfassend eine DNA-Sequenz, die zur Initiierung der Transkription durch Hefe-RNA-Polymerase II fähig ist und aus der Regulonregion eines der GAPDH-Gene von *S. cerevisiae* abgeleitet ist und im wesentlichen aus Polynukleotid -850 bis -1 von Fig. 2 besteht, nämlich

```
-850        -840        -830        -820        -810        -800
GAATTCCTCA  GTTTCAAGAT  CTTTTAATGT  CCAAAACCAT  TTGAGCCGAT  CTAAATACTT
-790        -780        -770        -760        -750        -740
CTGTGTTTTC  ATTAATTTAT  AAATTGTACT  CTTTTAAGAC  ATGGAAAGTA  CCAACATCGG
-730        -720        -710        -700        -690        -680
TTGAAACAGT  TTTTCATTTA  CATATGGTTT  ATTGGTTTTT  CCAGTGAATG  ATTATTTGTC
-670        -660        -650        -640        -630        -620
GTTACCCTTT  CGTAAAACTT  CAAACACGTT  TTTAAGTATT  GTTTACTTGC  TCTTTCGACA
-610        -600        -590        -580        -570        -560
TATATGATTA  TCCCTGCGCC  GCTAAAGTTA  AAGATGCAAA  AAACAGAAGA  CAACTGAAGT
-550        -540        -530        -520        -510        -500
TAATTTACGT  CAATTAAGTT  TTCCAGGGTA  ATGATGTTTT  GGGCTTCCAC  TAATTCAATA
-490        -480        -470        -460        -450        -440
AGTATGTCAT  GAAATACGTT  GTGAAGAGCA  TCCAGAAATA  ATGAAAAGAA  ACAACGAAAC
-430        -420        -410        -400        -390        -380
TGGGTCGGCC  TGTTGTTTCT  TTTCTTTACC  ACGTGATCTG  CGGCATTTAC  AGGAAGTCGC
-370        -360        -350        -340        -330        -320
GCGTTTTGCG  CAGTTGTTGC  AACGCAGCTA  CGGCTAACAA  AGCCTACTGG  AACTCGACTG
-310        -300        -290        -280        -270        -260
ATGTGTTAGG  GCCTAAAACT  GGTGGTGACA  GCTGAAGTGA  ACTATTCAAT  CCAATCATGT
-250        -240        -230        -220        -210        -200
CATGGCTGTC  ACAAAGACCT  TGCCGACCGC  ACGTACGAAC  ACATACGTAT  GCTAATATGT
-190        -180        -170        -160        -150        -140
GTTTTGATAC  TACCCAGTGA  TCGCAGACCT  GCAATTTTTT  TGTAGGTTTG  GAAGAATATA
-130        -120        -110        -100        -90         -80
TAAAGGTTGC  ACTCATTCAA  GATAGTTTTT  TTCTTGTGTG  TCTATTCATT  TTATTATTGT
-70         -60         -50         -40         -30         -20
TTGTTTAAAT  GTTAAAAAAA  CCAAGAACTT  AGTTTCAAAT  TAAATTCATC  ACACAAACAA
-10         -1
ACAAAACAAA
```

worin das Regulon wahlweise modifiziert ist, um mindestens eine Restriktionsenzym-Spaltstelle zu enthalten, um die Manipulation der Nukleotidsequenzregion für die Initiation der Proteinsynthese zu erleichtern.

2. DNA-Sequenz, die in ein rekombinantes DNA-Plasmid oder in ein Hefechromosom inseriert werden kann, umfassend:

(a) ein DNA-Sequenz gamäß Anspruch 1, die zur Initiation der Transkription durch Hefe-RNA-Polmerase II fähig ist, und

(b) ein oder mehrere Strukturgene, die von den GAPDH-Genen von *S. cerevisiae* verschieden sind, und mindestens zwei der Bestandteile (c)-(f)

(c) eine oder mehrere spezifische DNA-Sequenzen, die zur Termination der Transkription durch Hefe-RNA-Polymerase II fähig sind und die Polyadenylierung der mRNA bewirken können, und/oder

(d) ein oder mehrere Selektionsmarker, und/oder

(e) eine oder mehrere Nukleotidsequenzen, die die stabile Insertion in ein Chromosom von Hefen erlauben, oder eine oder mehrere DNA-Sequenzen, die die Replikation in Hefen, die zur Art *Saccharomyces* oder *Klyveromyces* gehören, regulieren, und/oder

(f) eine DNA-Sequenz, die ein Signalpolypeptid von nicht mehr als 30 Aminosäuren kodiert, das die Translokation von Proteinen unterstützt, wobei die DNA-Sequenz stromaufwärts von und in dem gleichen Leserahmen wie das Strukturgen angeordnet ist.

3. DNA-Sequenz gemäß Anspruch 2, dadurch gekennzeichnet, daß das Strukturgen von (b) Thaumatin oder Chymosin oder deren verschiedene allelische oder modifizierte Formen, wobei die modifizierten Formen die süß schmeckenden Eigenschaften von Thaumatin bzw. die Milch gerinnenden Eigenschaften von Chymosin nicht beeinträchtigen, oder Vorstufen dieser thaumatinartigen oder chymosinartigen Proteine kodiert.

4. DNA-Sequenz gemäß Anspruch 2, dadurch gekennzeichnet, daß die spezifische DNA-Sequenz von (c) eine DNA-Sequenz ist, die sowohl zur Termination der Transkription durch Hefe-RNA-Polymerase II fähig ist und auch die Polyadenylierung der mRNA bewirken kann, wobei die DNA-Sequenz aus der Terminations/Polyadenylierungs-Region abgeleitet ist, die zu einem der GAPDH-Gene in *S. cerevisiae* gehört und mindestens ein die *Hpa* I-Stelle enthaltendes *Afl* II-*Bam* HI-Fragment A umfaßt, das im wesentlichen die Formel der Polynukleotide 15—687 von Fig. 3 hat, nämlich

```
                          17         27         37         47         57
                         GGT   TACTTTAATG  ATTTACTTTT  TATTATTAAT  AATTCATGCT
             67          77         87         97        107        117
        CATGACATCT  CATATACACG  TTTATAAAAC  TTAAATAGAT  TGAAAATGTA  TTAAAGATTC
            127        137        147        157        167        177
        CTCAGGGATT  CGATTTTTTT  GGAAGTTTTT  GTTTTTTTTT  CCTTGAGATG  CTGTAGTATT
            187        197        207        217        227        237
        TGGGAACAAT  TATACAATCG  AAAGATATAT  GCTTACATTC  GACCGTTTTA  GCCGTGATCA
            247        257        267        277        287        297
        TTATCCTATA  GTAACATAAC  CTGAAGTATA  ACTGACACTA  CTATCATCAA  TACTTGTCAC
            307        317        327        337        347        357
        ATGAGAACTC  TGTGAATAAT  TAGGCCACTG  AAATTTGATG  CCTGAAGGAC  CGGCATCACG
            367        377        387        397        407        417
        TATCTTCGAT  AAAGCACTTA  GTATCACACT  AATTGGCTTT  TCGCCGCATA  TGGTGTTTCC
            427        437        447        457        467        477
        GGTGATTTCC  AAGTATTGTT  TCCAAGCATC  GTACCTTTCA  CCATTTGGAG  TATCACTTAG
            487        497        507        517        527        537
        CGTTTTCATC  GCATATCTGT  CCATTATTTC  AATGGATTGC  CAAATGGGAA  CTTGATGATG
            547        557        567        577        587        597
        TGAAAGTTTA  CTCCTAGCAG  TTAACATTTC  CACTTCTGTT  TCCTCTTTAA  TGGCATTCAT
            607        617        627        637        647        657
        TCAACTCTTC  CTTGCTTACC  GACGTACCCG  TATATTGGAA  TCTGCGGCCC  CAATGACAGA
            667        677        687
        AATCACTGCT  TACAATGAAT  AAATTGTTCG.
```

5. DNA-Sequenz gemäß Anspruch 2, dadurch gekennzeichnet, daß die ein Signalpolypeptid kodierende DNA-Sequenz von (f) ausgewählt ist aus der Gruppe von DNA-Sequenzen, die

(a) das Signalpolypeptid, das *S. cerevisiae* Invertase transloziert, nämlich Met.Leu.Leu.Gln.Ala.Phe.Leu.Phe.Leu.Leu.Ala.Gly.Phe.Ala.Ala.Lys.Ile.Ser.Ala;

(b) das Signalpolypeptid, das saure Phosphatase von *S. cerevisiae* transloziert, nämlich Met.Phe.Lys.Ser.Val.Val.Tyr.Ser.Ile.Leu.Ala.Ala.Ser.Leu.Ala.Asn.Ala;

(c) das Signalpolypeptid unreifer Formen von thaumatinartigen Proteinen, nämlich Met.Ala.Ala.Thr.Thr.Cys.Phe.Phe.Phe.Leu.Phe.Pro.Phe.Leu.Leu.Leu.Leu.Thr.Leu.Ser.Arg.Ala;

(d) das Signalpolypeptid unreifer Formen von chymosinartigen Proteinen, nämlich Met.Arg.Cys.Leu.Val.Val.Leu.Leu.Ala.Val.Phe.Ala.Leu.Ser.Gln.Gly; und

(e) zwei Consensus-Signalpolypeptide, nämlich
Met.Ser.Lys.Ala.Ala.Leu.Ala.Phe.Ile.Ala.Phe.Val.Ile.Val.Leu.Ile.Val.Asn.Ala und
Met.Ser.Lys.Phe.Val.Ile.Val.Leu.Ile.Val.Ala.Ala.Leu.Ala.Phe.Ile.Ala.Asn.Ala, kodieren.

6. DNA-Sequenz gemäß Anspruch 2, dadurch gekennzeichnet, daß entweder die Kodons des Strukturgens von (b) oder die Kodons der das Signalpolypeptid kodierenden DNA-Sequenz von (f) oder beide in von Hefen bevorzugt Kodons modifiziert werden.

7. DNA-Sequenz gemäß Anspruch 6, dadurch gekennzeichnet, daß als von Hefen bevorzugt Kodons die folgenden Kodons verwendet werden:

| Kodon | kodierend | Kodon | kodierend |
|---|---|---|---|
| GCC oder GCT | Alanin | TTG | Leucin |
| AGA | Arginin | AAG | Lysin |
| AAC | Asparagin | ATG | Methionin |
| GAC oder GAT | Asparaginsäure | TTC | Phenylalanin |
| TGT | Cystein | CCA | Prolin |
| CAA | Glutamin | TCC oder TCT | Serin |
| GAA | Glutaminsäure | ACC oder ACT | Threonin |
| GGT | Glycin | TGG | Tryptophan |
| CAC | Histidin | TAC | Tyrosin |
| ATC oder ATT | Isoleucin | GTC oder GTT | Valin. |

8. Verfahren zur Produktion von rDNA-Sequenzen enthaltenden Hefen, dadurch gekennzeichnet, daß DNA-Sequenzen gemäß Anspruch 2 in die Hefen *Saccharomyces*, *Kluyveromyces*, *Hansenula*, *Candida* oder *Torulopsis* entweder in Form von Plasmiden oder durch Einbau in das Hefegenom eingeführt werden.

9. Hefe, enthaltend eine rDNA-Sequenz gemäß Anspruch 2, entweder in Form eines Plasmids oder in das Hefegenom eingebaut.

10. Verfahren zur Produktion eines Proteins durch Kultur einer rDNA-Sequenzen enthaltenden Hefe, dadurch gekennzeichnet, daß eine Hefe gemäß Anspruch 9 zur Produktion eines Proteins verwendet wird, wobei die rDNA-Sequenz ein dieses Protein oder eine Vorstufe desselben kodierendes Strukturgen enthält, wobei die Vorstufe während der Prozessierung das relevante Protein bilden kann.

11. Verfahren gemäß Anspruch 10, in welchem das Protein ein thaumatinartiges Protein ist.

12. Verfahren gemäß Anspruch 10, in welchem das Protein ein chymosinartiges Protein ist.

**Patentansprüche für den Vertragsstaat: AT**

1. Verfahren zur Herstellung einer eine rDNA-Sequenz enthaltenden Hefe, dadurch gekennzeichnet, daß eine DNA-Sequenz, die in ein rekombinates DNA-Plasmid oder ein Hefechromosom inseriert werden kann, umfassend

(a) eine DNA-Sequenz, die eine verbesserte Expression der neu eingeführten Gene in Hefezellen ergibt, umfassend eine DNA-Sequenz, die zur Initiierung der Transkription durch Hefe-RNA-Polymerase II fähig ist und aus der Regulonregion eines der GAPDH-Gene von *S. cerevisiae* abgeleitet ist und im wesentlichen aus Polynukleotid -850 bis -1 von Fig. 2 besteht, nämlich

```
      -850         -840          -         -830         -820         -810         -800
      GAATTCCTCA  GTTTCAAGAT  CTTTTAATGT  CCAAAACCAT  TTGAGCCGAT  CTAAATACTT
      -790         -780         -770         -760         -750         -740
      CTGTGTTTTC  ATTAATTTAT  AAATTGTACT  CTTTTAAGAC  ATGGAAAGTA  CCAACATCGG
      -730         -720         -710         -700         -690         -680
      TTGAAACAGT  TTTTCATTTA  CATATGGTTT  ATTGGTTTTT  CCAGTGAATG  ATTATTTGTC
      -670         -660         -650         -640         -630         -620
      GTTACCCTTT  CGTAAAACTT  CAAACACGTT  TTTAAGTATT  GTTTAGTTGC  TCTTTCGACA
      -610         -600         -590         -580         -570         -560
      TATATGATTA  TCCCTGCGCG  GCTAAAGTTA  AAGATGCAAA  AAACAGAAGA  CAACTGAAGT
      -550         -540         -530         -520         -510         -500
      TAATTTACGT  CAATTAAGTT  TTCCAGGGTA  ATGATGTTTT  GGGCTTCCAC  TAATTCAATA
      -490         -480         -470         -460         -450         -440
      AGTATGTCAT  GAAATACGTT  GTGAAGAGCA  TCCAGAAATA  ATGAAAAGAA  ACAACGAAAC
      -430         -420         -410         -400         -390         -380
      TGGGTCGGCC  TGTTGTTTCT  TTTCTTTACC  ACGTGATCTG  CGGCATTTAC  AGGAAGTCGC
      -370         -360         -350         -340         -330         -320
      GCGTTTTGCG  CAGTTGTTGC  AACGCAGCTA  CGGCTAACAA  AGCCTAGTGG  AACTCGACTG
      -310         -300         -290         -280         -270         -260
      ATGTGTTAGG  GCCTAAAACT  GGTGGTGACA  GCTGAAGTGA  ACTATTCAAT  CCAATCATGT
      -250         -240         -230         -220         -210         -200
      CATGGCTGTC  ACAAAGACCT  TGCGGACCGC  ACGTACGAAC  ACATACGTAT  GCTAATATGT
      -190         -180         -170         -160         -150         -140
      GTTTTGATAC  TACCCAGTGA  TCGCAGACCT  GCAATTTTTT  TGTAGGTTTG  GAAGAATATA
      -130         -120         -110         -100         -90          -80
      TAAAGGTTGC  ACTCATTCAA  GATAGTTTTT  TTCTTGTGTG  TCTATTCATT  TTATTATTGT
      -70          -60          -50          -40          -30          -20
      TTGTTTAAAT  GTTAAAAAAA  CCAAGAACTT  AGTTTCAAAT  TAAATTCATC  ACACAAACAA
      -10          -1
      ACAAAACAAA
```

worin das Regulon wahlweise modifiziert ist, um mindestens eine Restriktionsenzym-Spaltstelle zu enthalten, um die Manipulation der Nukleotidsequenzregion für die Initiation der Proteinsynthese zu erleichtern, und

(b) ein oder mehrere Strukturgenene, die von den GAPDH-Genen von *S. cerevisiae* verschieden sind, und mindestens zwei der Bestandteile (c)-(f)

(c) eine oder mehrere spezifische DNA-Sequenzen, die zur Termination der Transkription durch Hefe-RNA-Polymerase II fähig sind und die Polyadenylierung der mRNA bewirken können, und/oder

(d) ein oder mehrere Selektionsmarker, und/oder

(e) entweder eine oder mehrere Nukleotidsequenzen, die die stabile Insertion in ein Chromosom von Hefen erlauben, oder eine oder mehrere DNA-Sequenzen, die die Replikation in Hefen, die zur Art *Saccharomyces* oder *Kluyveromyces* gehören, regulieren, und/oder

(f) eine DNA-Sequenz, die ein Signalpolypeptid von nicht mehr als 30 Aminosäuren kodiert, das die Translokation von Proteinen unterstützt, wobei die DNA-Sequenz stromaufwärts von und in dem gleichen Leserahmen wie das Strukturgen angeordnet ist, in Saccharomyces-, Kluyveromyces-, Hansenula-, Candida- oder torulopsis-Hefen, entweder in Form eines Plasmids oder durch Einverleibung in das Hefegenom, eingeführt wird.

2. Verfahren gemäß Anspruch 1, dadurch gekennzeichnet, daß das Strukturgen von (b) Thaumatin oder Chymosin oder deren verschiedene allelische oder modifizierte Formen, wobei die modifizierten Formen die süß schmeckenden Eigenschaften von Thaumatin bzw. die Milch gerinnenden Eigenschaften von Chymosin nicht beeinträchtigen, oder Vorstufen dieser thaumatinartigen oder chymosinartigen Proteine kodiert.

3. Verfahren gemäß Anspruch 1, dadurch gekennzeichnet, daß die spezifische DNA-Sequenz von (c) eine DNA-Sequenz ist, die sowohl zur Termination der Transkription durch Hefe-RNA-Polymerase II fähig ist und auch die Polyadenylierung der mRNA bewirken kann, wobei die DNA-Sequenz aus der Terminations/Polyadenylierungs-Region abgeleitet ist, die zu einem der GAPDH-Gene in *S. cerevisiae* gehört und mindestens ein die *Hpa* I-Stelle enthaltendes *Afl* II-*Bam* HI-Fragment A umfaßt, das im wesentlichen die Formel der Polynukleotide 15—687 von Fig. 3 hat, nämlich

```
                    17          27          37          47          57
                    GGT  TACTTTAATG  ATTTACTTTT  TATTATTAAT  AATTCATGCT
        67          77          87          97          107         117
CATGACATCT  CATATACACG  TTTATAAAAC  TTAAATAGAT  TGAAAATGTA  TTAAAGATTC
        127         137         147         157         167         177
CTCAGGGATT  CGATTTTTTT  GGAAGTTTTT  GTTTTTTTTT  CCTTGAGATG  CTCTAGTATT
        187         197         207         217         227         237
TGGGAACAAT  TATACAATCG  AAAGATATAT  GCTTACATTC  GACCGTTTTA  GCCGTGATCA
        247         257         267         277         287         297
TTATCCTATA  GTAACATAAC  CTGAAGTATA  ACTGACACTA  CTATCATCAA  TACTTGTCAC
        307         317         327         337         347         357
ATGAGAACTC  TGTGAATAAT  TAGGCCACTG  AAATTTGATG  CCTGAAGGAC  CGGCATCACG
        367         377         387         397         407         417
TATCTTCGAT  AAAGCACTTA  GTATCACACT  AATTGGCTTT  TCGCCGCATA  TGGTGTTTCC
        427         437         447         457         467         477
GGTGATTTCC  AAGTATTGTT  TCCAAGCATC  GTACCTTTCA  CCATTTGGAG  TATCACTTAG
        487         497         507         517         527         537
CGTTTTCATC  GCATATCTGT  CCATTATTTC  AATGGATTGC  CAAATGGGAA  CTTGATGATG
        547         557         567         577         587         597
TGAAAGTTTA  CTCCTAGCAG  TTAACATTTC  CACTTCTGTT  TCCTCTTTAA  TGGCATTCAT
        607         617         627         637         647         657
TCAACTCTTC  CTTGCTTACC  GACGTACCCG  TATATTGGAA  TCTGCGGCCC  CAATGACAGA
        667         677         687
AATCACTGCT  TACAATGAAT  AAATTGTTCG.
```

4. Verfahren gemäß Anspruch 1, dadurch gekennzeichnet, daß die ein Signalpolypeptid kodierende DNA-Sequenz von (f) ausgewählt ist aus der Gruppe von DNA-Sequenzen, die

(a) das Signalpolypeptid, das *S. cerevisiae* Invertase transloziert, nämlich Met.Leu.Leu.Gln.Ala.Phe.Leu.Phe.Leu.Leu.Ala.Gly.Phe.Ala.Ala.Lys.Ile.Ser.Ala;

(b) das Signalpolypeptid, das saure Phosphatase von *S. cerevisiae* transloziert, nämlich Met.Phe.Lys.Ser.Val.Val.Tyr.Ser.Ile.Leu.Ala.Ala.Ser.Leu.Ala.Asn.Ala;

(c) das Signalpolypeptid unreifer Formen von thaumatinartigen Proteinen, nämlich Met.Ala.Ala.Thr.Thr.Cys.Phe.Phe.Phe.Leu.Phe.Pro.Phe.Leu.Leu.Leu.Leu.Thr.Leu.Ser.Arg.Ala;

(d) das Signalpolypeptid unreifer Formen von chymosinartigen Proteinen, nämlich Met.Arg.Cys.Leu.Val.Val.Leu.Leu.Ala.Val.Phe.Ala.Leu.Ser.Gln.Gly; und

(e) zwei Consensus-Signalpolypeptide, nämlich Met.Ser.Lys.Ala.Ala.Leu.Ala.Phe.Ile.Ala.Phe.Val.Ile.Val.Leu.Ile.Val.Asn.Ala und Met.Ser.Lys.Phe.Val.Ile.Val.Leu.Ile.Val.Ala.Ala.Leu.Ala.Phe.Ile.Ala.Asn.Ala, kodieren.

5. Verfahren gemäß Anspruch 1, dadurch gekennzeichnet, daß entweder die Kodons des Strukturgens von (b) oder die Kodons der das Signalpolypeptid kodierenden DNA-Sequenz von (f) oder beide in von Hefen bevorzugt Kodons modifiziert werden.

6. Verfahren gemäß Anspruch 5, dadurch gekennzeichnet, daß als von Hefen bevorzugte Kodons die folgenden Kodons verwendet werden:

| Kodon | kodierend | Kodon | kodierend |
|---|---|---|---|
| GCC oder GCT | Alanin | TTG | Leucin |
| AGA | Arginin | AAG | Lysin |
| AAC | Asparagin | ATG | Methionin |
| GAC oder GAT | Asparaginsäure | TTC | Phenylalanin |
| TGT | Cystein | CCA | Prolin |
| CAA | Glutamin | TCC oder TCT | Serin |
| GAA | Glutaminsäure | ACC oder ACT | Threonin |
| GGT | Glycin | TGG | Tryptophan |
| CAC | Histidin | TAC | Tyrosin |
| ATC oder ATT | Isoleucin | GTC oder GTT | Valin. |

7. Verfahren zur Produktion eines Proteins durch Kultur einer rDNA-Sequenzen enthaltenden Hefe, dadurch gekennzeichnet, daß eine Hefe, hergestellt gemäß irgendeinem der Ansprüche 1 bis 6, oder ihre Nachkommenschaft zur Produktion eines Proteins verwendet wird, wobei die rDNA-Sequenz ein dieses Protein oder eine Vorstufe desselben kodierendes Strukturgen enthält, wobei die Vorstufe während der Prozessierung das relevante Protein bilden kann.

8. Verfahren gemäß Anspruch 7, in welchem das Protein ein thaumatinartiges Protein ist.

9. Verfahren gemäß Anspruch 7, in welchem das Protein ein chymosinartiges Protein ist.

**Revendications pour les Etats contractants: BE CH DE FR GB IT NL SE LI**

1. Séquence d'ADN assurant une expression améliorée de gènes nouvellement introduits dans des cellules de levure, compenant une séquence d'ADN capable d'initier la transcription par l'ARN polymérase II de levure, laquelle dernière séquence d'ADN dérive de la région régulon d'un des gènes GAPDH de S. Cerevisiae et consiste essentiellement en le polynucléotide −850 à -1 de la figure 2, c'est-à-dire

```
-850        -840        -830        -820        -810        -800
GAATTCCTCA  GTTTCAAGAT  CTTTTAATGT  CCAAAACCAT  TTGAGCCGAT  CTAAATACTT
-790        -780        -770        -760        -750        -740
CTGTGTTTTC  ATTAATTIAT  AAATTGTACT  CTTTTAAGAC  ATGGAAAGTA  CCAACATCGG
-730        -720        -710        -700        -690        -680
TTGAAACAGT  TTTTCATTTA  CATATGGTTT  ATTGGTTTTT  CCAGTGAATG  ATTATTTGTC
-670        -660        -650        -640        -630        -620
GTTACCCTTT  CGTAAAACTT  CAAACACGTT  TTTAAGTATT  GTTTAGTTGC  TCTTTCGACA
-610        -600        -590        -580        -570        -560
TATATGATTA  TCCCTGCGCG  GCTAAAGTTA  AAGATGCAAA  AAACAGAAGA  CAACTGAAGT
-550        -540        -530        -520        -510        -500
TAATTTACGT  CAATTAAGTT  TTCCAGGGTA  ATGATGTTTT  GGGCTTCCAC  TAATTCAATA
-490        -480        -470        -460        -450        -440
AGTATGTCAT  GAAATACGTT  GTGAAGAGCA  TCCAGAAATA  ATGAAAGAA   ACAACGAAAC
-430        -420        -410        -400        -390        -380
TGGGTCGGCC  TGTTGTTTCT  TTTCTTTACC  ACGTGATCTG  CGGCATTTAC  AGGAAGTCGC
-370        -360        -350        -340        -330        -320
GCGTTTTGCG  CAGTTGTTGC  AACGCAGCTA  CGGCTAACAA  AGCCTAGTGG  AACTCGACTG
-310        -300        -290        -280        -270        -260
ATGTGTTAGG  GCCTAAAACT  GGTGGTGACA  GCTGAAGTGA  ACTATTCAAT  CCAATCATGT
-250        -240        -230        -220        -210        -200
CATGGCTGTC  ACAAAGACCT  TGCGGACCGC  ACGTACGAAC  ACATACGTAT  GCTAATATGT
-190        -180        -170        -160        -150        -140
GTTTTGATAG  TACCCAGTGA  TCGCAGACCT  GCAATTTTTT  TGTAGGTTTG  GAAGAATATA
-130        -120        -110        -100        -90         -80
TAAACGTTGC  ACTCATTCAA  GATAGTTTTT  TTCTTGTGTG  TCTATTCATT  TTATTATTGT
-70         -60         -50         -40         -30         -20
TTGTTTAAAT  GTTAAAAAAA  CCAAGAACTT  AGTTTCAAAT  TAAATTCATC  ACACAAACAA
-10         -1
ACAAAACAAA
```

où le régulon est facultativement modifié pour comprendre au moins un site de clivage par une enzyme de restriction, pour faciliter la manipulation de la région de séquence de nucléotides pour l'initiation de la synthèse des protéines.

2. Séquence d'ADN qui peut être insérée dans un plasmide à ADN recombinant ou dans un chromosome de levure, comprenant:

(a) une séquence d'ADN selon la revendication 1, capable d'initier la transcription par l'ARN polymérase II de levure et

(b) un ou plusieurs gènes de structure différents des gènes GAPDH de S. cerevisiae et au moins deux des caractéristiques (c)-(f),

(c) une ou plusieurs séquences d'ADN spécifiques capables de terminer la transcription par l'ARN polymérase II de levure et d'effectuer une polyadénylation de l'ARNm, et/ou

(d) un ou plusieurs marqueurs de sélection, et/ou

(e) soit un ou plusieurs séquences de nucléotides permettant l'insertion stable dans un chromosome de levures, soit une ou plusieurs séquences d'ADN qui régulent la réplication dans les levures appartenant au genre *Saccharomyces* ou au genre *Kluyveromyces*, et/ou

(f) une séquence d'ADN codant pour un polypeptide-signal n'ayant pas plus de 30 restes d'amino-acide contribuant à la translocation des protéines, laquelle séquences d'ADN est située en amont du gène de structure et dans le même cadre de lecture.

3. Séquence d'ADN selon la revendication 2, caractérisée en ce que le gène de structure de (b) code pour la thaumatine ou la chymosine ou leurs diverses formes alléliques ou modifiées, lesquelles formes modifiées ne présentent pas de détérioration repectivement des propriétés sucrantes de la thaumatine ou des propriétés de coagulation du lait de la chymosine, ou des précurseurs de ces protéines de type thaumatine ou de type chymosine.

4. Séquence d'ADN selon la revendication 2, caractérisée en ce que la séquence d'ADN spécifique de (c) est une séquence d'ADN capable à la fois d'assurer l'arrêt de la transcription par l'ARN polymérase II de levure et d'effectuer la polyadénylation de l'ARNm, laquelle séquence d'ADN dérive de la région de terminaison/polyadénylation appartenant à un des génes GAPDH de *S. cerevisiae* et comprend au moins le fragment A Afl II-BamH I contenant le site Hpa I ayant essentiellement la formule du polynucléotide 15—687 de le figure 3, c'est-à-dire

```
               17         27         37         47         57
               GGT   TACTTTAATG ATTTACTTTT TATTATTAAT AATTCATGCT
        67         77         87         97        107        117
   CATGACATCT CATATACACG TTTATAAAAC TTAAATAGAT TGAAAATGTA TTAAAGATTC
       127        137        147        157        167        177
   CTCAGGGATT CGATTTTTTT GGAAGTTTTT GTTTTTTTTT CCTTGAGATG CTGTAGTATT
       187        197        207        217        227        237
   TGGGAACAAT TATACAATCG AAAGATATAT GCTTACATTC GACCGTTTTA GCCGTGATCA
       247        257        267        277        287        297
   TTATCCTATA GTAACATAAC CTGAAGTATA ACTGACACTA CTATCATCAA TACTTGTCAC
       307        317        327        337        347        357
   ATGAGAACTC TGTCAATAAT TAGCCCACTG AAATTTGATG CCTGAAGGAC CGGCATCACG
       367        377        387        397        407        417
   TATCTTCGAT AAAGCACTTA GTATCACACT AATTGGCTTT TCGCCGCATA TGGTGTTTCC
       427        437        447        457        467        477
   GGTGATTTCC AAGTATTGTT TCCAAGCATC GTACCTTTCA CCATTTGGAG TATCACTTAG
       487        497        507        517        527        537
   CGTTTTCATC GCATATCTGT CCATTATTTC AATGGATTGC CAAATGGGAA CTTGATGATG
       547        557        567        577        587        597
   TGAAAGTTTA CTCCTAGCAG TTAACATTTC CACTTCTGTT TCCTCTTTAA TGGCATTCAT
       607        617        627        637        647        657
   TCAACTCTTC CTTGCTTACC GACGTACCCG TATATTGGAA TCTGCGGCCC CAATGACAGA
       667        677        687
   AATCACTGCT TACAATGAAT AAATTGTTCG.
```

5. Séquence d'ADN selon la revendication 2, caractérisée en ce que la séquence d'ADN de (f) codant pour un polypeptide-signal est choisie dans le groupe constitué des séquences d'ADN codant pour

(a) le polypeptide-signal de translocation de l'invertase de *S. cerevisiae*, c'est-à-dire Met.Leu.Leu.Gln.Ala.Phe.Leu.Phe.Leu.Leu.Ala.Gly.Phe.Ala.Ala.Lys.Ile.Ser.Ala;

(b) le polypeptide-signal de translocation de la phosphatase acide de *S. cerevisiae*, c'est-à-dire Met.Phe.Lys.Ser.Val.Val.Tyr.Ser.Ile.Leu.Ala.Ala.Ser.Leu.Ala.Asn.Ala;

(c) le polypeptide-signal des formes n'ayant pas subi de maturation des protéines de type thaumatine, c'est-à-dire Met.Ala.Ala.Thr.Thr.Cys.Phe.Phe.Phe.Leu.Phe.Pro.Phe.Leu.Leu.Leu.Leu.Thr.Leu.Ser.Arg.Ala;

(d) le polypeptide-signal des formes n'ayant pas subi de maturation des protéines de type chymosine, c'est-à-dire Met.Arg.Cys.Leu.Val.Val.Leu.Leu.Ala.Val.Phe.Ala.Leu.Ser.Gln.Gly; et

(e) deux polypeptide-signal consensus, c'est-à-dire
Met.Ser.Lys.Ala.Ala.Leu.Ala.Phe.Ile.Ala.Phe.Val.Ile.Val.Leu.Ile.Val.Asn.Ala et
Met.Ser.Lys.Phe.Val.Ile.Val.Leu.Ile.Val.Ala.Ala.Leu.Ala.Phe.Ile.Ala.Asn.Ala.

6. Séquence d'ADN selon la revendication 2, caractérisée en ce que, soit les codons du gène de structure de (b), soit les codons de la séquence d'ADN codant pour un polypeptide-signal de (f), soit les deux, sont modifiés en codons préférés par les levures.

7. Séquence d'ADN selon la revendication 6, caractérisée en ce que, comme codons préférés par les levures, on utilise les codons suivants:

| Codon | Code pour | Codon | Code pour |
|---|---|---|---|
| GCC ou GCT | alanine | TTG | leucine |
| AGA | arginine | AAG | lysine |
| AAC | asparagine | ATG | méthionine |
| GAC ou GAT | acide aspartique | TTC | phénylalanine |
| TGT | cystéine | CCA | proline |
| CAA | glutamine | TCC ou TCT | sérine |
| GAA | acide glutamique | ACC ou ACT | thréonine |
| GGT | glycine | TGG | tryptophane |
| CAC | histidine | TAC | tyrosine |
| ATC ou ATT | isoleucine | GTC ou GTT | valine |

8. Procédé pour préparer des levures contenant des séquences d'ADNr, caractérisé en ce que les séquences d'ADN selon la revendication 2 sont introduites dans les levures *Saccharomyces*, *Kluyveromyces*, *Hansenula*, *Candida* ou *Torulopsis*, soit sous forme de plasmides, soit par incorporation au génome de la levure.

9. Levure contenant une séquence d'ADNr selon la revendication 2, soit sous forme d'un plasmide, soit incorporée au génome de la levure.

10. Procédé pour préparer une protéine par culture d'une levure contenant des séquences d'ADNr, caractérisée en ce qu'une levure, selon la revendication 9, est utilisée pour produire une protéine, la séquence d'ADNr contenant un gène de structure codant pour cette protéine ou pour un précurseur de celle-ci, lequel précurseur, au cours de la mise en oeuvre, peut former la protéine désirée.

11. Procédé selon la revendication 10, dans lequel la protéine est une protéine de type thaumatine.

12. Procédé selon la revendication 10, dans lequel la protéine est une protéine de type chymosine.

**Revendications pour l'Etat contractant: AT**

1. Procédé pour préparer une levure contenant une séquence d'ADNr, caractérisée en ce qu'une séquence d'ADN, qui peut être insérée dans un plasmide à ADN recombinant ou dans un chromosome de levure, comprenant:

(a) une séquence d'ADN assurant une expression améliorée de gènes nouvellement introduits dans des cellules de levure, comprenant une séquence d'ADN capable d'initier la transcription par l'ARN polymérase II de levure, laquelle dernière séquence d'ADN dérive de la région régulon d'un des gènes GAPDH de *S. cerevisiae* et consiste essentiellement en le polynucléotide −850 à -1 de la figure 2, c'est-à-dire

```
          -850        -840        -830        -820        -810        -800
          GAATTCCTCA  GTTTCAAGAT  CTTTTAATGT  CCAAAACCAT  TTGAGCCGAT  CTAAATACTT
          -790        -780        -770        -760        -750        -740
          CTCTGTTTTC  ATTAATTTAT  AAATTGTACT  CTTTTAAGAC  ATGGAAAGTA  CCAACATCGG
          -730        -720        -710        -700        -690        -680
          TTGAAACAGT  TTTTCATTTA  CATATGGTTT  ATTGGTTTTT  CCAGTGAATG  ATTATTTGTC
          -670        -660        -650        -640        -630        -620
          GTTACCCTTT  CGTAAAACTT  CAAACACGTT  TTTAAGTATT  GTTAGTTGC   TCTTTCGACA
          -610        -600        -590        -580        -570        -560
          TATATGATTA  TCCCTGCGCG  GCTAAAGTTA  AAGATGCAAA  AAACAGAAGA  CAACTGAAGT
          -550        -540        -530        -520        -510        -500
          TAATTTACGT  CAATTAACTT  TTCCAGGGTA  ATGATGTTTT  GGGCTTCCAC  TAATTCAATA
          -490        -480        -470        -460        -450        -440
          AGTATGTCAT  GAAATACGTT  GTGAAGAGCA  TCCAGAAATA  ATGAAAAGAA  ACAACGAAAC
          -430        -420        -410        -400        -390        -380
          TGGGTCGGCC  TGTTGTTTCT  TTTCTTTACC  ACGTGATCTG  CGGCATTTAC  ACGAAGTCGC
          -370        -360        -350        -340        -330        -320
          CGGTTTTGCG  CAGTTGTTGC  AACGCAGCTA  CGGCTAACAA  AGCCTAGTGG  AACTCGACTG
          -310        -300        -290        -280        -270        -260
          ATGTGTTAGG  GCCTAAAACT  GGTGGTGACA  GCTGAAGTGA  ACTATTCAAT  CCAATCATGT
          -250        -240        -230        -220        -210        -200
          CATGGCTGTC  ACAAAGACCT  TGCGGACCGC  ACGTACGAAC  ACATACGTAT  GCTAATATGT
          -190        -180        -170        -160        -150        -140
          GTTTGATAG   TACCCAGTGA  TCGCAGACCT  GCAATTTTTT  TGTAGGTTTG  GAAGAATATA
          -130        -120        -110        -100        -90         -80
          TAAAGGTTGC  ACTGATTCAA  GATAGTTTTT  TTCTTGTGTG  TCTATTCATT  TTATTATTGT
          -70         -60         -50         -40         -30         -20
          TTGTTTAAAT  GTTAAAAAAA  CCAAGAACTT  AGTTTCAAAT  TAAATTCATC  ACACAAACAA
          -10         -1
          ACAAAACAAA
```

où le régulon est facultativement modifié pour comprendre au moins un site de clivage par une enzyme de restriction, pour faciliter la manipulation de la région de séquence de nucléotides pour l'initiation de la synthèse des protéines, et

(b) un ou plusieurs gènes de structure différents des gènes GAPDH de *S. cerevisiae* et au moins deux des caractéristiques (c)-(f),

(c) une ou plusieurs séquences d'ADN spécifiques capables de terminer la transcription par l'ARN polymérase II de levure et d'effectuer une polyadénylation de l'ARNm, et/ou

(d) un ou plusieurs marqueurs de sélection, et/ou

(e) soit un ou plusieurs séquences de nucléotides permettant l'insertion stable dans un chromosome de levures, soit une ou plusieurs séquences d'ADN qui régulent la réplication dans les levures appartenant au genre *Saccharomyces* ou au genre *Kluyveromyces*, et/ou

(f) une séquence d'ADN codant pour un polypeptide-signal n'ayant pas plus de 30 restes d'aminoacides contribuant à la translocation des protéines, laquelle séquences d'ADN est située en amont du gène de structure et dans le même cadre de lecture, est introduite dans les levures *Saccharomyces*, *Kluyveromyces*, *Hansenula*, *Candida*, ou *Torulopsis* soit sous forme d'un plasmide, soit par incorporation au génome de la levure.

2. Procédé selon la revendication 1, caractérisée en ce que le gène de structure de (b) code pour la thaumatine ou la chymosine ou leurs diverses formes alléliques ou modifiées, lesquelles formes modifiées ne présentent pas de détérioration repectivement des propriétés sucrantes de la thaumatine ou des propriétés de coagulation du lait de la chymosine, ou des précurseurs de ces protéines de type thaumatine ou de type chymosine.

3. Procédé selon la revendication 1, caractérisée en ce que la séquence d'ADN spécifique de (c) est une séquence d'ADN capable à la fois d'assurer l'arrêt de la transcription par l'ARN polymérase II de levure et d'effectuer la polyadénylation de l'ARNm, laquelle séquence d'ADN dérive de la région de terminaison/ polyadénylation appartenant à un des génes GAPDH de *S. cerevisiae* et comprend au moins le fragment A Afl II-BamH I contenant le site Hpa I ayant essentiellement la formule du polynucléotide 15—687 de le figure 3, c'est-à-dire

```
                    17          27          37          47          57
                    GGT TACTTTAATG ATTTACTTTT TATTATTAAT AATTCATGCT
           67  .         77          87          97         107         117
     CATGACATCT CATATACACG TTTATAAAAC TTAAATAGAT TGAAAATCTA TTAAAGATTC
          127         137         147         157         167         177
     CTCAGGGATT CGATTTTTTT GGAAGTTTTT GTTTTTTTTT CCTTGAGATG CTGTAGTATT
          187         197         207         217         227         237
     TGGGAACAAT TATACAATCG AAAGATATAT GCTTACATTC GACCGTTTTA GCCGTGATCA
          247         257         267         277         287         297
     TTATCCTATA GTAACATAAC CTGAAGTATA ACTGACACTA CTATCATCAA TACTTGTCAC
          307         317         327         337         347         357
     ATGAGAACTC TGTGAATAAT TAGGCCACTG AAATTTGATG CCTGAAGGAC CGGCATCACG
          367         377         387         397         407         417
     TATCTTCGAT AAAGCACTTA GTATCACACT AATTGGCTTT TCGCCGCATA TGGTGTTTCC
          427         437         447         457         467         477
     GGTGATTTCC AAGTATTGTT TCCAAGCATC GTACCTTTCA CCATTTGGAG TATCACTTAG
          487         497         507         517         527         537
     CGTTTTCATC GCATATCTGT CCATTATTTC AATGGATTGC CAAATGGGAA CTTGATGATG
          547         557         567         577         587  ·       597
     TGAAAGTTTA CTCCTAGCAG TTAACATTTC CACTTCTGTT TCCTCTTTAA TGGCATTCAT
          607         617         627         637         647         657
     TCAACTCTTC CTTGCTTACC GACGTACCCG TATATTGGAA TCTGCGGCCC CAATGACAGA
          667         677         687
     AATCACTGCT TACAATGAAT AAATTGTTCG.
```

4. Procédé selon la revendication 1, caractérisé ce que la séquence d'ADN de (f) codant pour un polypeptide-signal est choisie dans le groupe constitué des séquences d'ADN codant pour

(a) le polypeptide-signal de translocation de l'invertase de *S. cerevisiae*, c'est-à-dire Met.Leu.Leu.Gln.Ala.Phe.Leu.Phe.Leu.Leu.Ala.Gly.Phe.Ala.Ala.Lys.Ile.Ser.Ala;

(b) le polypeptide-signal de translocation de la phosphatase acide de *S. cerevisiae*, c'est-à-dire Met.Phe.Lys.Ser.Val.Val.Tyr.Ser.Ile.Leu.Ala.Ala.Ser.Leu.Ala.Asn.Ala;

(c) le polypeptide-signal des formes n'ayant pas subi de maturation des protéines de type thaumatine, c'est-à-dire Met.Ala.Ala.Thr.Thr.Cys.Phe.Phe.Phe.Leu.Phe.Pro.Phe.Leu.Leu.Leu.Leu.Thr.Leu.Ser.Arg.Ala;

(d) le polypeptide-signal des formes n'ayant pas subi de maturation des protéines de type chymosine, c'est-à-dire Met.Arg.Cys.Leu.Val.Val.Leu.Leu.Ala.Val.Phe.Ala.Leu.Ser.Gln.Gly; et

(e) deux polypeptide-signal consensus, c'est-à-dire
Met.Ser.Lys.Ala.Ala.Leu.Ala.Phe.Ile.Ala.Phe.Val.Ile.Val.Leu.Ile.Val.Asn.Ala et
Met.Ser.Lys.Phe.Val.Ile.Val.Leu.Ile.Val.Ala.Ala.Leu.Ala.Phe.Ile.Ala.Asn.Ala.

5. Procédé selon la revendication 1, caractérisé ce que soit les codons du gène de structure de (b), soit les codons de la séquence d'ADN codant pour un polypeptide-signal de (f), soit les deux, sont modifiés en codons préférés par les levures.

6. Procédé selon la revendication 5, caractérisée en ce que, comme codons préférés par les levures, on utilise les codons suivants:

| Codon | Code pour | Codon | Code pour |
|---|---|---|---|
| GCC ou GCT | alanine | TTG | leucine |
| AGA | arginine | AAG | lysine |
| AAC | asparagine | ATG | méthionine |
| GAC ou GAT | acide aspartique | TTC | phénylalanine |
| TGT | cystéine | CCA | proline |
| CAA | glutamine | TCC ou TCT | sérine |
| GAA | acide glutamique | ACC ou ACT | thréonine |
| GGT | glycine | TGG | tryptophane |
| CAC | histidine | TAC | tyrosine |
| ATC ou ATT | isoleucine | GTC ou GTT | valine |

7. Procédé pour préparer une protéine par culture d'une levure contenant des séquences d'ADNr, caractérisé en ce qu'une levure, préparée par un procédé selon l'une quelconque des revendications 1 à 6, ou sa descendance, est utilisée pour produire une protéine, la séquence d'ADNr contenant un gène de structure codant pour cette protéine ou pour un précurseur de celle-ci, lequel précurseur, au cours de la maturation moléculaire, peut former la protéine désirée.

8. Procédé selon la revendication 7, dans lequel la protéine est une protéine de type thaumatine.

9. Procédé selon la revendication 7, dans lequel la protéine est une protine de type chymosine.

Fig.1.

EP 0 129 268 B1

```
-850       -840       -830       -820       -810       -800
GAATTCCTCA GTTTCAAGAT CTTTTAATGT CCAAAACCAT TTGAGCCGAT CTAAATACTT
-790       -780       -770       -760       -750       -740
CTGTGTTTTC ATTAATTTAT AAATTGTACT CTTTTAAGAC ATGGAAAGTA CCAACATCGG
-730       -720       -710       -700       -690       -680
TTGAAACAGT TTTTCATTTA CATATGGTTT ATTGGTTTTT CCAGTGAATG ATTATTTGTC
-670       -660       -650       -640       -630       -620
GTTACCCTTT CGTAAAACTT CAAACACGTT TTTAAGTATT GTTTAGTTGC TCTTTCGACA
-610       -600       -590       -580       -570       -560
TATATGATTA TCCCTGCGCG GCTAAAGTTA AAGATGCAAA AAACAGAAGA CAACTGAAGT
-550       -540       -530       -520       -510       -500
TAATTTACGT CAATTAAGTT TTCCAGGGTA ATGATGTTTT GGGCTTCCAC TAATTCAATA
-490       -480       -470       -460       -450       -440
AGTATGTCAT GAAATACGTT GTGAAGAGCA TCCAGAAATA ATGAAAAGAA ACAACGAAAC
-430       -420       -410       -400       -390       -380
TGGGTCGGCC TGTTGTTTCT TTTCTTTACC ACGTGATCTG CGGCATTTAC AGGAAGTCGC
-370       -360       -350       -340       -330       -320
GCGTTTTGCG CAGTTGTTGC AACGCAGCTA CGGCTAACAA AGCCTAGTGG AACTCGACTG
-310       -300       -290       -280       -270       -260
ATGTGTTAGG GCCTAAAACT GGTGGTGACA GCTGAAGTGA ACTATTCAAT CCAATCATGT
-250       -240       -230       -220       -210       -200
CATGGCTGTC ACAAAGACCT TGCGGACCGC ACGTACGAAC ACATACGTAT GCTAATATGT
-190       -180       -170       -160       -150       -140
GTTTGATAG TACCCAGTGA TCGCAGACCT GCAATTTTTT TGTAGGTTTG GAAGAATATA
-130       -120       -110       -100       -90        -80
TAAAGGTTGC ACTCATTCAA GATAGTTTTT TTCTTGTGTG TCTATTCATT TTATTATTGT
-70        -60        -50        -40        -30        -20
TTGTTTAAAT GTTAAAAAAA CCAAGAACTT AGTTTCAAAT TAAATTCATC ACACAAACAA
-10        -1
ACAAAACAAA ATG
```

*Fig.2.*

*Fig.3.*

```
          7         17         27         37       •  47  •      57
TAAATTTAAC TCCTTAAGGT TACTTTAATG ATTTAGTTTT TATTATTAAT AATTCATGCT
          67         77         87         97        107        117
CATGACATCT CATATACACG TTTATAAAAC TTAAATAGAT TGAAAATGTA TTAAAGATTC
         127        137        147        157        167        177
CTCAGGGATT CGATTTTTTT GGAAGTTTTT GTTTTTTTTT CCTTGAGATG CTGTAGTATT
         187        197        207        217        227        237
TGGGAACAAT TATACAATCG AAAGATATAT GCTTACATTC GACCGTTTTA GCCGTGATCA
         247        257        267        277        287        297
TTATCCTATA GTAACATAAC CTGAAGTATA ACTGACACTA CTATCATCAA TACTTGTCAC
         307        317        327        337        347        357
ATGAGAACTC TGTGAATAAT TAGGCCACTG AAATTTGATG CCTGAAGGAC CGGCATCACG
         367        377        387        397        407        417
TATCTTCGAT AAAGCACTTA GTATCACACT AATTGGCTTT TCGCCGCATA TGGTGTTTCC
         427        437        447        457        467        477
GGTGATTTCC AAGTATTGTT TCCAAGCATC GTACCTTTCA CCATTTGGAG TATCACTTAG
         487        497        507        517        527        537
CGTTTTCATC GCATATCTGT CCATTATTTC AATGGATTGC CAAATGGGAA CTTGATGATG
         547        557        567        577        587        597
TGAAAGTTTA CTCCTAGCAG TTAACATTTC CACTTCTGTT TCCTCTTTAA TGGCATTCAT
         607        617        627        637      .  647        657
TCAACTCTTC CTTGCTTACC GACGTACCCG TATATTGGAA TCTGCGGCCC CAATGACAGA
         667        677    •    687      .  697        707        710
AATCACTGCT TACAATGAAT AAATTGTTCG GATCCTTAAT GTACTCCGAC AAAATATTAC
         727        737        747        757        767        777
CAATGCAACG ATCAACATCA ACGCTGTTAT GAGAAACCAT CATGGGAATT ACCTTCACCG
         787        797        807        817        827        837
TATCTAAAGA AATTTCTCTC CATTTCAAAG TTTCCACCAA CATGGGGAGC TGCATCTCTA
         847        857        867        877        887        897
AGGAATGTTC AGCCATATCA GTGTCATGAT CCATTGGCTT AAACAGCTTC TTTCCGTTCT
         907        917        927        937        947        957
CAGGATACTC CTTCTGTATT AATGTTTTAC ACAAGTCTGT ATCCACTTTC AGATTACCCA
         967        977        987        997       1007       1017
AGGGCGTCTC TAGCTCACTG AATGCACTAA CTAAAATTTG GTTTTTGAAA TAGATGTGAT
        1027       1037       1047       1057       1067       1077
GCGACGGCCC CAAGATAAAT ATTCTCTTAA CATTACGGTT CAAATCCAAC GATGCGTACG
        1087       1097       1107       1117       1127       1137
AGTAGGCCAT AGTGGGTCCA CAATACCTGT AACCGGCATG AGGACATATG ATAATTCTGG
        1147       1157       1167       1177       1187       1197
CGTTGTGAAT TGGGCCTTTA AGGGTACTTT TGATCAAGTA TGTATGCGGT TGTTGAGATA
        1207       1217       1227       1237       1247       1257
ATTCTTGGGC TCTATTGGAA TACCATGAGC CTGCATGTGT TGCTGGACGT ATTGACATGT
        1267       1277       1287       1297       1307       1317
TTGAAAAATT CTATTCTTTG CACTGTAGTC CACCTAAGCC ACCGACTAGG ACCACTTCAC
        1322
TTAAG
```

Fig .4.

*Fig.5.*

Fig.6.

## Fig.7a.

<u>Sac 1</u>

5' CCC.TTA.GTT.TCA.AAT.TAA.AGA.GCT.CAT.CAC 3'

        3' TCT.CGA.GTA.GTG.TGT.TTG.TTT.GTT.TTG.TTT 5'

            &darr; Klenow DNA-polymerase
               dNTP's

<u>Dde I</u>       <u>Sac I</u>

5' CCC.TTA.GTT.TCA.AAT.TAA.AGA.GCT.CAT.CAC.ACA.AAC.AAA.CAA.AAC.AAA 3'

3' GGG.AAT.CAA.AGT.TTA.ATT.TCT.CGA.GTA.GTG.TGT.TTG.TTT.GTT.TTG.TTT 5'

         &darr; Dde I

       <u>Sac I</u>

 5' TTA.GTT.TCA.AAT.TAA.AGA.GCT.CAT.CAC.ACA.AAC.AAA.CAA.AAC.AAA 3'

  3' CAA.AGT.TTA.ATT.TCT.CGA.GTA.GTG.TGT.TTG.TTT.GTT.TTG.TTT 5'

          Sac I

          T$_4$ DNA-polymerase, dNTP's

          T$_4$ DNA ligase

 5' TTA.GTT.TCA.AAT.TAA.AGC.ATC.ACA.CAA.ACA.AAC.AAA.ACA.AA 3'

  3' CAA.AGT.TTA.ATT.TCG.TAG.TGT.GTT.TGT.TTG.TTT.TGT.TT 5'

## Fig.7b.

5' A.GCT.CAT.CAC.ACA.AAC.AAA.CAA.AAC.AAA 3'

 3' TA.GTC.TGT.TTG.TTT.GTT.TTG.TTT 5'

Fig.8.

Fig.9.

## Fig.10.

EP 0 129 268 B1

# Fig.11.

1. H₂N.NH₂

2. Lev ... + MSNT

3. Ac₂O

4. Repeat cycle

AcO

LevO

B = A^Bz, C^An, G^DPA, T

Lev = CH₃COCH₂CH₂CO

(P) = Polystyrene support

Fig.12.

# Fig.13.

# Fig.14.

Fig.15.

Fig. 16.

Fig.17.

*Fig.18.*

Fig.19

*Fig. 20.*

# Fig. 21.

LEADER SEQUENCE PREPROCHYMOSIN IN S. CER. PREFERRED CODONS

```
    ATGAGATGTT TGGTTGTTTT GTTGGCTGTT TTCGCTTTGT CCCAAGGT
-174      -165       -155       -145       -135      -127
```

DNA SEQUENCE PRO PART PRECEEDING PSEUDOCHYMOSIN IN S. CER. PREFERRED CODONS

```
    GCTGAAATTA CTAGAATTCC ATTGTACAAG GGTAAGTCCT TGAGAAAGGC TTTGAAGGAA
-126       -116       -106       -96        -86        -76
    CACGGTTTGT TGGAAGACTT C
-66        -56        -46
```

DNA SEQUENCE PSEUDO PART PRECEEDING CHYMOSIN IN S. CER PREFERRED CODONS

```
    TTGCAAAAGC AACAATACGG TATTTCCTCC AAGTACTCCG GTTTC
-45       -36        -26        -16        -6    -1
```

DNA SEQUENCE CHYMOSIN IN S. CER. PREFERED CODONS

```
         10         20         30         40         50         60
GGTGAAGTTG CTTCCGTTCC ATTGACTAAC TACTTGGACT CCCAATACTT CGGTAAGATT
         70         80         90        100        110        120
TACTTGGGTA CTCCACCACA AGAATTCACT GTTTTGTTCG ACACTGGTTC CTCCGACTTC
        130        140        150        160        170        180
TGGGTTCCAT CCATTTACTG TAAGTCCAAC GCTTGTAAGA ACCACCAAAG ATTCGACCCA
        190        200        210        220        230        240
AGAAAGTCCT CCACTTTCCA AAACTTGGGT AAGCCATTGT CCATTCACTA CGGTACTGGT
        250        260        270        280        290        300
TCCATGCAAG GTATTTTGGG TTACGACACT GTTACTGTTT CCAACATTGT TGACATTCAA
        310        320        330        340        350        360
CAAACTGTTG GTTTGTCCAC TCAAGAACCA GGTGACGTTT TCACTTACGC TGAATTCGAC
        370        380        390        400        410        420
GGTATTTTGG GTATGGCTTA CCCATCCTTG GCTTCCGAAT ACTCCATTCC AGTTTTCGAC
        430        440        450        460        470        480
AACATGATGA ACAGACACTT GGTTGCTCAA GACTTGTTCT CCGTTTACAT GGACAGAAAC
        490        500        510        520        530        540
GGTCAAGAAT CCATGTTGAC TTTGGGTGCT ATTGACCCAT CCTACTACAC TGGTTCCTTG
        550        560        570        580        590        600
CACTGGGTTC CAGTTACTGT TCAACAATAC TGGCAATTCA CTGTTGACTC CGTTACTATT
        610        620        630        640        650        660
TCCGGTGTTG TTGTTGCTTG TGAAGGTGGT TGTCAAGCTA TTTTGGACAC TGGTACTTCC
        670        680        690        700        710        720
AAGTTGGTTG GTCCATCCTC CGACATTTTG AACATTCAAC AAGCTATTGG TGCTACTCAA
        730        740        750        760        770        780
AACCAATACG GTGAATTCGA CATTGACTGT GACAACTTGT CCTACATGCC AACTGTTGTT
        790        800        810        820        830        840
TTCGAAATTA ACGGTAAGAT GTACCCATTG ACTCCATCCG CTTACACTTC CCAAGACCAA
        850        860        870        880        890        900
GGTTTCTGTA CTTCCGGTTT CCAATCCGAA AACCACTCCC AAAAGTGGAT TTTGGGTGAC
        910        920        930        940        950        960
GTTTTCATTA GAGAATACTA CTCCGTTTTC GACAGAGCTA ACAACTTGGT TGGTTTGGCT
        969
AAGGCTATT
```

21

*Fig.22.*

LEADER SEQUENCE OF PREPROTHAUMATIN IN S.CER. PREFERRED CODONS

```
ATGGCTGCTA CTACTTGTTT CTTCTTCTTG TTCCCATTCT TGTTGTTGTT GACTTTGTCC
-66        -57        -47        -37        -27        -17        -7

AGAGCT
  -1
```

DNA SEQUENCE MATURE THAUMATIN IN S. CER. PREFERRED CODONS

```
          10         20         30         40         50         60
   GCTACTTTCG AAAATTGTTAA CAGATGTTCC TACACTGTCT GGGCTGCTGC TTCCAAGGGT
          70         80         90        100        110        120
   GACGCTGCTT TGGACGCTGG TGGTAGACAA TTGAACTCCG GTGAATCCTG GACTATTAAC
         130        140        150        160        170        180
   GTTGAACCAG GTACTAAGGG TGGTAAGATT TGGGCTAGAA CTGACTGTTA CTTCGACGAC
         190        200        210        220        230        240
   TCCGGTAGAG GTATTTGTAG AACTGGTGAC TGTGGTGGTT TGTTGCAATG TAAAGAGATTC
         250        260        270        280        290        300
   GGTAGACCAC CAACTACTTT GGCTGAATTC TCCTTGAACC AATACGGTAA GGACTACATT
         310        320        330        340        350        360
   GACATTTCCA ACATTAAGGG TTTCAACGTT CCAATGTACT TCTCCCCAAC TACTAGAGGT
         370        380        390        400        410        420
   TGTAGAGGTG TTAGATGTGC TGCTGACATT GTTGGTCAAT GTCCAGCTAA GTTGAAGGCT
         430        440        450        460        470        480
   CCAGGTGGTG GTTGTAACGA CGCTTGTACT GTTTTCCAAA CTTCCGAATA CTGTTGTACT
         490        500        510        520        530        540
   ACTGGTAAGT GTGGTCCAAC TGAATACTCC AGATTCTTCA AGAGATTGTG TCCAGACGCT
         550        560        570        580        590        600
   TTCTCCTACG TTTTGGACAA GCCAACTACT GTTACTTGTC CAGGTTCCTC CAACTACAGA
         610        620
   GTTACTTTCT GTCCAACTGC T
```

DNA SEQUENCE ACIDIC PEPTIDE OF PROTHAUMATIN IN S.CER. PREFERRED CODONS

```
622        632
TTGGAATTGG AAGACGAA
```

LEADER SEQUENCE ACIDIC PHOSPHATASE IN YEAST PREFERRED CODONS

ATGTTCAAGT CCGTTGTTTA CTCCATTTTG GCTGCTTCCT TGGCTAACGC T
-51          -41          -31          -21          -11          -1


LEADER SEQUENCE INVERTASE IN YEAST PREFERED CODONS

ATGTTGTTGC AAGCTTTCTT GTTCTTGTTG GCTGGTTTCG CTGCTAAGAT TTCCGCT
-57          -47          -37          -27          -17          -7    -1

*Fig.23.*

## Fig. 24.

Prochymosin

Pro-    Pseudo-                    Chymosin

−126   −46        I                                                969   *
        −45   −1

Leader sequences-encoding DNA sequences

Leader sequences used are those originating from:
Pre Acid phosphatase   (−1 to −51   or −4 to −54*, Fig. 23)
Pre Invertase          (−1 to −54   or −4 to −54 , Fig. 23)
Pre Pro Thaumatin       (−1 to −66, Fig 22)
and two consensus leader sequences (Fig. 25, −1 to −57).

* nucleotides number according to previous figures.

## Fig.25.

CONSENSUS LEADER SEQUENCE (A-B) IN YEAST PREFERRED CODONS

```
ATGTCCAAGG CTGCTTTGGC TTTCATTGCT TTCGTTATTG TTTTGATTGT TAACGCT
    -48         -38        -28        -18        -8       -1
```

CONSENSUS LEADER SEQUENCE (B-A) IN YEAST PREFERRED CODONS

```
ATGTCCAAGT TCGTTATTGT TTTGATTGTT GCTGCTTTGG CTTTCATTGC TAACGCT
     -48         -38        -28        -18        -8      -1
```